# EUROPEAN PATENT APPLICATION

(11) **EP 4 681 744 A1**
(43) Date of publication of application: **21.01.2026**
(21) Application number: 24912666.5
(22) Date of filing: 20.12.2024
(51) Int. Cl.: A61L 31/04, A61P 9/00, A61P 11/00, A61P 25/00, A61P 43/00, C12N 11/08

(54) **SHEET FOR REPAIRING NERVE INJURY, SHEET FOR REPAIRING BRAIN INJURY, AND SHEET FOR REPAIRING SPINAL CORD INJURY**

(30) Priority: 26.12.2023 JP 2023219794
(71) Applicant: Tama Bio Co., Ltd., Musashino-shi, Tokyo 180-0022 (JP); Tohoku University, Aoba-ku Sendai-shi Miyagi 980-8577 (JP)
(72) Inventor: NIIZUMA Kuniyasu, Sendai-shi, Miyagi 980-8577 (JP); OSAWA Shinichiro, Sendai-shi, Miyagi 980-8577 (JP); ANDO Daisuke, Sendai-shi, Miyagi 980-8577 (JP); TOMINAGA Keita, Sendai-shi, Miyagi 980-8577 (JP); NAKAYASHIKI Atsushi, Sendai-shi, Miyagi 980-8577 (JP); NAGAO Tetsuya, Musashino-shi, Tokyo 180-0022 (JP)
(74) Representative: van Trier, Norbertus Henricus Gerardus
(86) International application number: PCT/JP2024/045181
(87) International publication number: WO 2025/142777

(57) **Abstract**

The present invention provides a biological tissue regenerative treatment sheet for regenerating biological tissue, a nerve damage repair sheet, a brain damage repair sheet, a spinal cord damage repair sheet, a lung damage repair sheet, a peritoneal damage repair sheet, a blood vessel damage repair sheet, a culture sheet and regenerating treatment method.

The biological tissue regenerative treatment sheet comprises a first surface including a roughened surface to be brought into contact with a site of nerve damage and phagocytosed by in vivo tissue cells; and a second surface disposed opposite to the first surface. The roughened surface comprises polytetrafluoroethylene as a main component.

## Description

### Technical Field

The present invention relates to sheets for regenerative treatment of biological tissue, sheets for repairing nerve damage, sheets for repairing brain damage, sheets for repairing spinal cord damage, sheets for repairing lung damage, sheets for repairing peritoneal damage, sheets for repairing blood vessel damage, culture sheets, and regenerative treatment methods, among others.

### Background Art

Various materials are used for dura mater reconstruction in neurosurgical operations. While expanded polytetrafluoroethylene, one of the materials for artificial dura mater, is a stable material, it has poor biocompatibility. For example, expanded polytetrafluoroethylene has poor adhesion to autologous dura mater. Therefore, in the biological repair material described in Patent Document1, biocompatibility is improved by subjecting an expanded polytetrafluoroethylene sheet to ion beam irradiation.

More specifically, in the biological repair material described in Patent Document 1, fibrin glue is combined with expanded polytetrafluoroethylene, at least a part of the surface of which has been modified with ion bombardment by ion implantation.

Expanded polytetrafluoroethylene has been applied to dura mater defects. However, this application is solely for the purpose of compensating for the dura mater defect (in other words, supplementing the defect with the artificial material), and regeneration of the biological tissue at the defect site is not envisioned.

### Prior Art Documents

### Patent Literature

Patent Literature 1:Japanese Patent No. 4445697

### Summary of the Invention

### Problems to be Solved by the Invention

An object of the present invention is to provide a sheet for regenerative treatment of biological tissue that regenerates biological tissue, a sheet for repairing nerve damage, a sheet for repairing brain damage, a sheet for repairing spinal cord damage, a sheet for repairing lung damage, a sheet for repairing peritoneal damage, a sheet for repairing blood vessel damage, a culture sheet, and a regenerative treatment method.

### Means for Solving the Problems

Hereinafter, means for solving the problems will be described using the numbers and symbols used in the embodiments for carrying out the invention. These numbers and symbols are added in parentheses for reference to indicate an example of the correspondence between the claims and the embodiments for carrying out the invention. Therefore, the claims should not be restrictively interpreted by the description in parentheses.

A biological tissue regenerative treatment sheet in some embodiments comprises a first surface (20) including a roughened surface (20r) that will be phagocytosed by cells of biological tissue, and a second surface (30) disposed opposite to the first surface (20). The roughened surface (20r) contains polytetrafluoroethylene as a main component.

In the above biological tissue regenerative treatment sheet, the roughened surface (20r) may be constituted by the surface of an ion-implanted layer (2).

The above biological tissue regenerative treatment sheet may be configured such that the cells of the biological tissue can infiltrate the interior of the ion-implanted layer (2).

In the above biological tissue regenerative treatment sheet, the roughened surface (20r) may have a depression (20d) approximately the same size as the cells that phagocytose the roughened surface (20r).

The above biological tissue regenerative treatment sheet may be configured such that when the roughened surface (20r) is placed in contact with in vivo tissue (4), new tissue develops from the in vivo tissue (4) along the roughened surface (20r), and the new tissue interlocks with the irregularities of the roughened surface (20r).

The above biological tissue regenerative treatment sheet may be configured such that when the roughened surface (20r) is placed in contact with damaged in vivo tissue (4), the expression of at least one of mesenchymal stem cells, fibroblasts, myofibroblasts, macrophages, and oligodendrocytes is more prominent compared to when the second surface (30) is placed in contact with the damaged in vivo tissue (4).

The above biological tissue regenerative treatment sheet may be configured such that when the roughened surface (20r) is placed in contact with damaged in vivo tissue (4), the expression of mesenchymal stem cells is more prominent compared to when the second surface (30) is placed in contact with the damaged in vivo tissue (4).

The above biological tissue regenerative treatment sheet may be configured such that when the roughened surface (20r) is placed in contact with damaged in vivo tissue (4), capillaries (B) are newly formed along the roughened surface (20r).

The above biological tissue regenerative treatment sheet may be configured such that when the roughened surface (20r) is placed in contact with the edge (4e) of the in vivo tissue (4) such that the roughened surface (20r) faces the defective region (RG) of the in vivo tissue (4), a biological membrane (5) and capillaries (B) are newly formed along the roughened surface (20r) in the defective region (RG).

The above biological tissue regenerative treatment sheet may be configured such that when the roughened surface (20r) is placed in contact with the in vivo tissue (4) for six months or more, the opaque polytetrafluoroethylene becomes transparent or semi-transparent.

A nerve damage repair sheet in some embodiments includes a first surface (20) including a roughened surface (20r) that will be phagocytosed by cells of in vivo tissue (4), and a second surface (30) disposed opposite to the first surface (20). The roughened surface (20r) contains polytetrafluoroethylene as a main component.

The above nerve damage repair sheet may be configured such that nerve cells are regenerated using the roughened surface (20r) as a scaffold when the roughened surface (20r) is placed to face a nerve damage site (RG1) of the in vivo tissue (4).

A brain damage repair sheet in some embodiments includes a first surface (20) including a roughened surface (20r) that will be phagocytosed by cells of in vivo tissue (4), and a second surface (30) disposed opposite to the first surface (20). The roughened surface (20r) contains polytetrafluoroethylene as a main component.

A spinal cord damage repair sheet in some embodiments includes a first surface (20) including a roughened surface (20r) that will be phagocytosed by cells of in vivo tissue (4), and a second surface (30) disposed opposite to the first surface (20). The roughened surface (20r) contains polytetrafluoroethylene as a main component.

A lung damage repair sheet in some embodiments includes a first surface (20) including a roughened surface (20r) that will be phagocytosed by cells of in vivo tissue (4), and a second surface (30) disposed opposite to the first surface (20). The roughened surface (20r) contains polytetrafluoroethylene as a main component.

A peritoneal damage repair sheet in some embodiments includes a first surface (20) including a roughened surface (20r) that will be phagocytosed by cells of in vivo tissue (4), and a second surface (30) disposed opposite to the first surface (20). The roughened surface (20r) contains polytetrafluoroethylene as a main component.

A blood vessel damage repair sheet in some embodiments includes a first surface (20) including a roughened surface (20r) that will be phagocytosed by cells of in vivo tissue (4), and a second surface (30) disposed opposite to the first surface (20). The roughened surface (20r) contains polytetrafluoroethylene as a main component.

A culture sheet in some embodiments includes a first surface (20) including a roughened surface (20r) on which target cells (C) for culture or biological tissue containing the cells (C) are placed, and a second surface (30) disposed opposite to the first surface (20). The roughened surface (20r) contains polytetrafluoroethylene as a main component.

A regenerative treatment method in some embodiments is a regenerative treatment method for humans or animals other than humans. The regenerative treatment method includes a step of preparing a biological tissue regenerative treatment sheet (1A) having a first surface (20) at least partially composed of a roughened surface (20r) containing polytetrafluoroethylene as a main component, and a second surface (30) disposed opposite to the first surface (20); a step of placing the biological tissue regenerative treatment sheet (1A) on damaged in vivo tissue (4) such that the roughened surface (20r) is in contact with the damaged in vivo tissue (4); and a step of regenerating the tissue at the damaged site of the in vivo tissue (4) using the roughened surface (20r) as a scaffold. The step of regenerating the tissue at the damaged site of the in vivo tissue (4) includes the cells of the in vivo tissue (4) phagocytosing at least a portion of the roughened surface (20r).

### Advantageous Effects of the Invention

According to the present invention, it is possible to provide a biological tissue regenerative treatment sheet that regenerates biological tissue, a nerve damage repair sheet, a brain damage repair sheet, a spinal cord damage repair sheet, a lung damage repair sheet, a peritoneal damage repair sheet, a blood vessel damage repair sheet, a culture sheet, and a regenerative treatment method.

### Brief Description of the Drawings

[Fig. 1] Fig. 1 is a schematic diagram showing a part of the biological tissue regenerative treatment sheet according to the first embodiment.
[Fig. 2] Fig. 2 is a schematic perspective view schematically showing the biological tissue regenerative treatment sheet according to the first embodiment.
[Fig. 3] Fig. 3 is a schematic cross-sectional view showing an enlarged part of the biological tissue regenerative treatment sheet according to the first embodiment.
[Fig. 4] Fig. 4 is a photograph substituting for a drawing showing the state in which the head of a rat was incised.
[Fig. 5] Fig. 5 is a photograph substituting for a drawing showing the state in which the head of a rat has undergone craniotomy.
[Fig. 6] Fig. 6 is a photograph substituting for a drawing showing the state in which the biological tissue regenerative treatment sheet according to the first embodiment was placed so as to cover the craniotomy site.
[Fig. 7] Fig. 7 is a photograph substituting for a drawing showing the state in which the biological tissue regenerative treatment sheet was covered with autologous bone and bone wax. [Fig. 8] Fig. 8 is a photograph substituting for a drawing showing the state in which the biological tissue regenerative treatment sheet according to the first embodiment has become semi-transparent and a pale yellow transparent biological membrane has been formed around the biological tissue regenerative treatment sheet.
[Fig. 9] Fig. 9 is a photograph substituting for a drawing showing the state in which the biological tissue regenerative treatment sheet according to the first embodiment was placed so as to close the opening of the peritoneum.
[Fig. 10] Fig. 10 is a photograph substituting for a drawing showing the state in which a semi-transparent biological membrane was formed along the biological tissue regenerative treatment sheet according to the first embodiment, and capillaries B were newly formed inside the biological membrane.
[Fig. 11] Fig. 11 is a photograph substituting for a drawing showing the state in which a biological membrane was newly formed so as to interlock with the roughened surface 20r of the biological tissue regenerative treatment sheet 1A.
[Fig. 12] Fig. 12 is a photograph substituting for a drawing showing a phagocytic image of ePTFE.
[Fig. 13] Fig. 13 is an electron micrograph of the roughened surface side of the biological tissue regenerative treatment sheet.
[Fig. 14] Fig. 14 is a photograph substituting for a drawing showing the state in which the second surface of the biological tissue regenerative treatment sheet and the biological membrane are separated.
[Fig. 15] Fig. 15 is a photograph substituting for a drawing showing a culture target.
[Fig. 16] Fig. 16 is a micrograph showing the state of cells cultured in MSC medium after 0 days.
[Fig. 17] Fig. 17 is a micrograph showing the state of cells cultured in MSC medium after 2 days.
[Fig. 18] Fig. 18 is a micrograph showing the state of cells cultured in MSC medium after 7 days.
[Fig. 19] Fig. 19 is a micrograph about staining of each marker.
[Fig. 20] Fig. 20 is a photograph substituting for a drawing showing the results of scRNA-seq analysis.
[Fig. 21] Fig. 21 is a photograph substituting for a drawing showing the results of scRNA-seq analysis.
[Fig. 22] Fig. 22 is a schematic cross-sectional view schematically showing the state in which the biological tissue regenerative treatment sheet according to the first embodiment was placed in biological tissue.
[Fig. 23] Fig. 23 is a schematic cross-sectional view schematically showing the state in which a biological membrane was newly formed along the roughened surface of the biological tissue regenerative treatment sheet according to the first embodiment.
[Fig. 24] Fig. 24 is a schematic cross-sectional view schematically showing the state in which the biological tissue regenerative treatment sheet according to the first embodiment was placed on a brain damage site.
[Fig. 25] Fig. 25 is a schematic cross-sectional view schematically showing the state in which the biological tissue regenerative treatment sheet according to the first embodiment was placed on a spinal cord damage site.
[Fig. 26] Fig. 26 is a schematic cross-sectional view schematically showing the state in which the biological tissue regenerative treatment sheet according to the first embodiment was placed on a damaged site of a lung structure.
[Fig. 27] Fig. 27 is a schematic cross-sectional view schematically showing the state in which the biological tissue regenerative treatment sheet according to the first embodiment was placed on a damaged site of a lung structure.
[Fig. 28] Fig. 28 is a schematic cross-sectional view schematically showing the state in which the biological tissue regenerative treatment sheet according to the first embodiment was placed on a damaged site of a blood vessel.
[Fig. 29] Fig. 29 is a schematic cross-sectional view schematically showing the state in which the biological tissue regenerative treatment sheet according to the first embodiment was placed on a damaged site of a blood vessel.
[Fig. 30] Fig. 30 is a schematic cross-sectional view schematically showing the state in which cells are cultured using the culture sheet.
[Fig. 31] Fig. 31 is a flowchart showing an example of a regenerative treatment method according to the second embodiment.
[Fig. 32] Fig. 32 is a photograph showing the state of placement of the biological tissue regenerative treatment sheet 1A in Example 1.
[Fig. 33] Fig. 33 is a graph showing the results of the rotarod test in Example 1.
[Fig. 34] Fig. 34 is a diagram for explaining the corner test in Example 1.
[Fig. 35] Fig. 35 is a graph showing the results of the corner test in Example 1.
[Fig. 36] Fig. 36(a) is a photograph showing the state of the biological tissue regenerative treatment sheet 1A placed for the roughened surface outer group in Example 1 after 5.5 months. Fig. 36(b) is a photograph showing the state of the biological tissue regenerative treatment sheet 1A placed for the roughened surface inner group in Example 1 after 5.5 months.
[Fig. 37] Fig. 37 is a photograph showing the state of cells stained with an MSC marker in Example 3.
[Fig. 38] Fig. 38 is a graph showing the results of single-cell RNA sequencing in Example 4. [Fig. 39] Fig. 39 is a diagram for explaining that mesenchymal stem cells are involved in anti-inflammatory action.

### Description of Embodiments

Hereinafter, a biological tissue regenerative treatment sheet, a nerve damage repair sheet, a brain damage repair sheet, a spinal cord damage repair sheet, a lung damage repair sheet, a peritoneal damage repair sheet, a blood vessel damage repair sheet, a culture sheet, and a regenerative treatment method according to the embodiments will be described with reference to the drawings. In the following description, members and parts having the same function are denoted by the same reference numerals, and redundant description of members and parts denoted by the same reference numerals will be omitted.

### First Embodiment

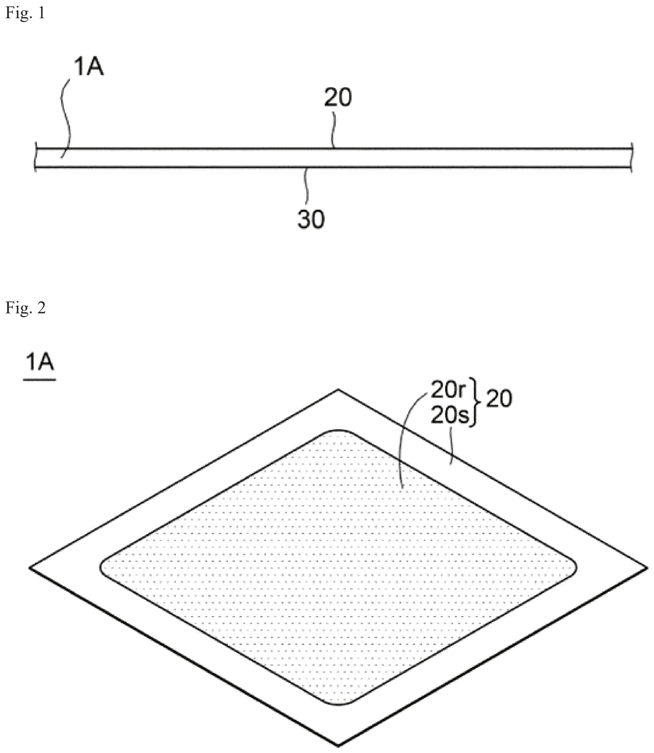

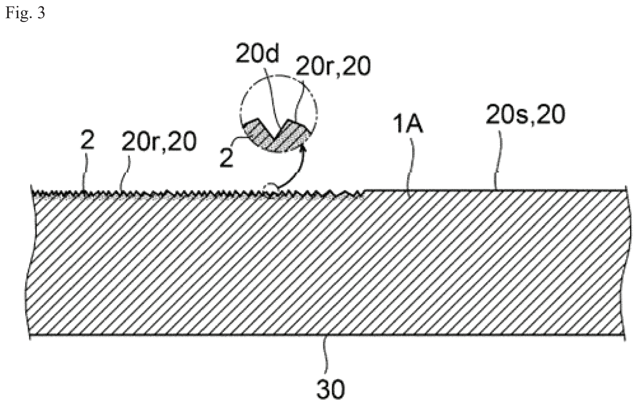

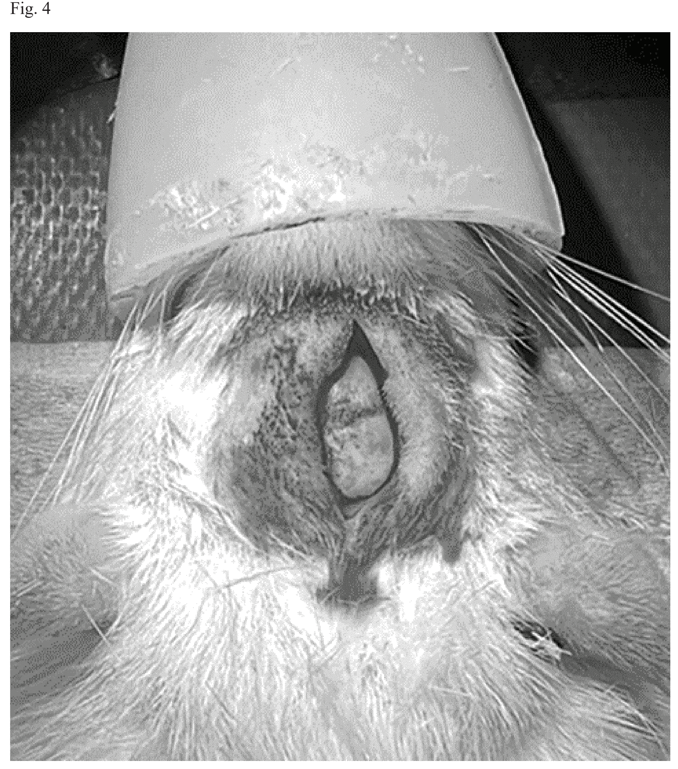

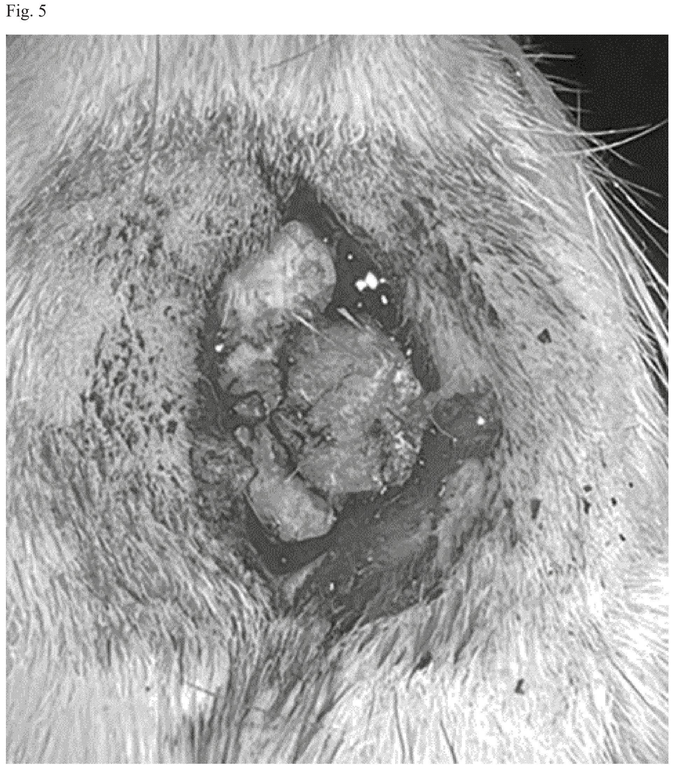

The biological tissue regenerative treatment sheet 1A according to the first embodiment will be described with reference to FIGS. 1 to 30. FIG. 1 is a schematic diagram schematically showing a part of the biological tissue regenerative treatment sheet 1A according to the first embodiment. FIG. 2 is a schematic perspective view schematically showing the biological tissue regenerative treatment sheet 1A according to the first embodiment. FIG. 3 is a schematic cross-sectional view showing an enlarged part of the biological tissue regenerative treatment sheet 1A according to the first embodiment. FIG. 4 is a photograph substituting for a drawing showing the state in which the head of a rat was incised. FIG. 5 is a photograph substituting for a drawing showing the state in which the head of a rat has undergone craniotomy.

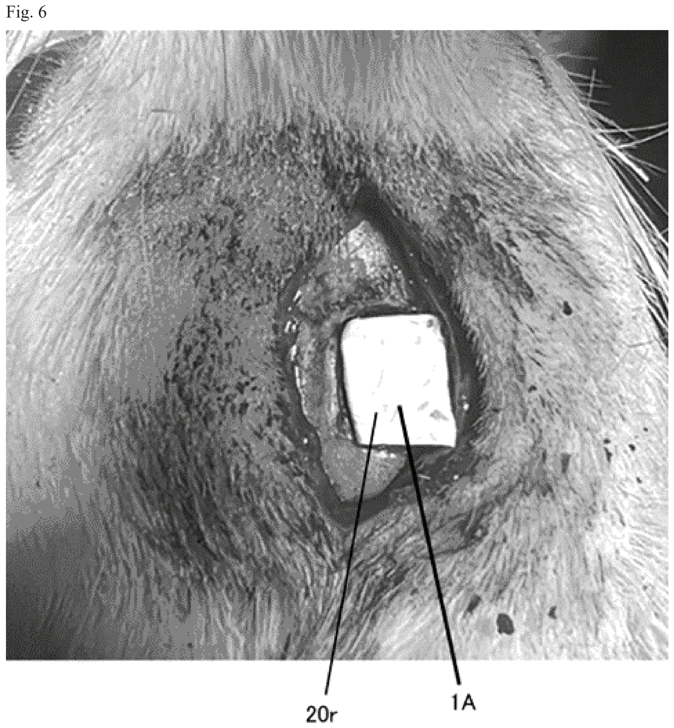

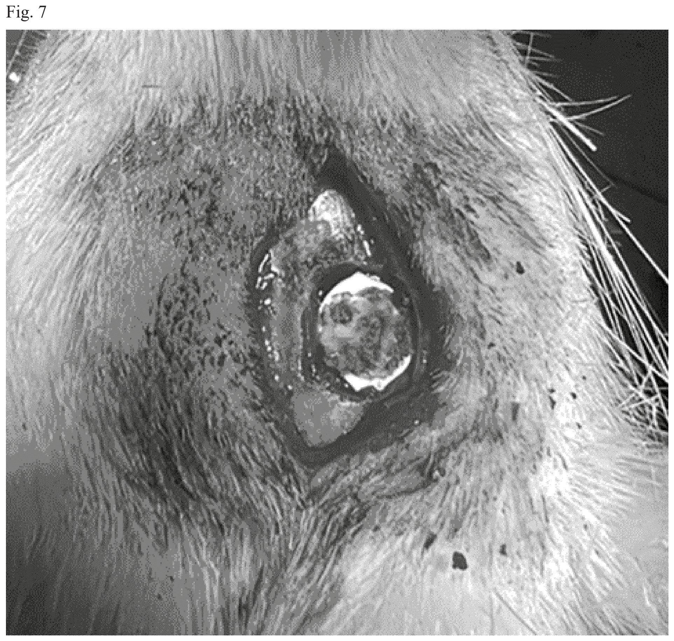

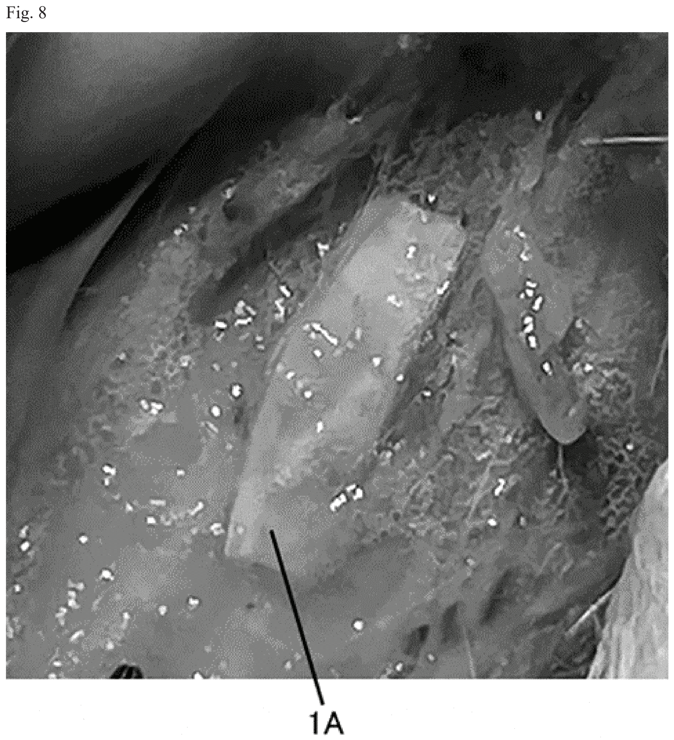

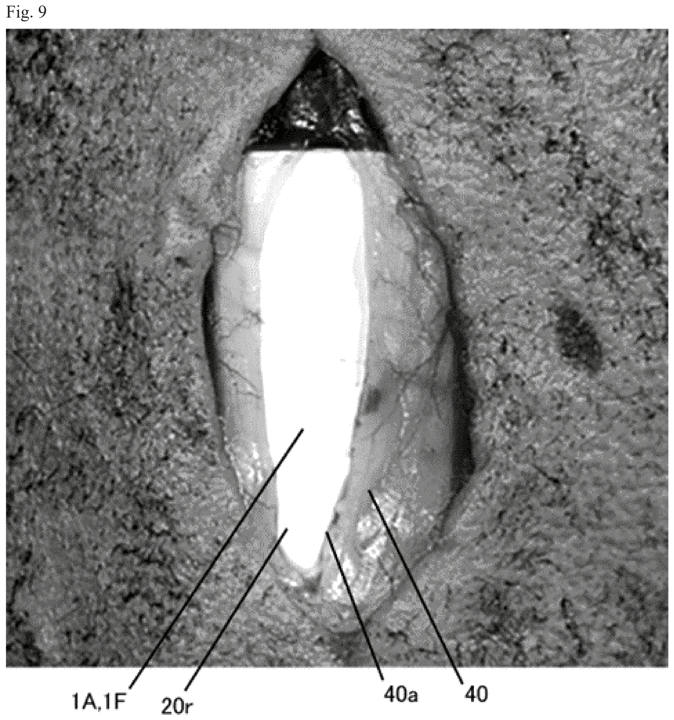

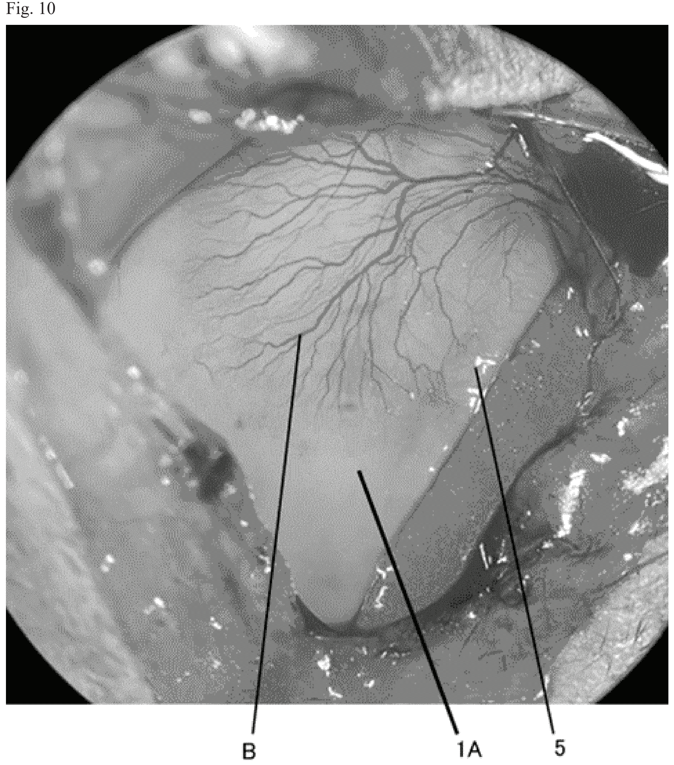

FIG. 6 is a photograph substituting for a drawing showing the state in which the biological tissue regenerative treatment sheet 1A according to the first embodiment was placed so as to cover the craniotomy site. FIG. 7 is a photograph substituting for a drawing showing the state in which the biological tissue regenerative treatment sheet 1A was covered with autologous bone and bone wax. FIG. 8 is a photograph substituting for a drawing showing the state in which the biological tissue regenerative treatment sheet 1A according to the first embodiment has become semi-transparent and a pale yellow transparent biological membrane has been formed around the biological tissue regenerative treatment sheet 1A. FIG. 9 is a photograph substituting for a drawing showing the state in which the biological tissue regenerative treatment sheet 1A according to the first embodiment was placed so as to close the opening 40a of the peritoneum 40. FIG. 10 is a photograph substituting for a drawing showing the state in which a semi-transparent biological membrane 5 was formed along the biological tissue regenerative treatment sheet 1A according to the first embodiment, and capillaries B were newly formed inside the biological membrane 5.

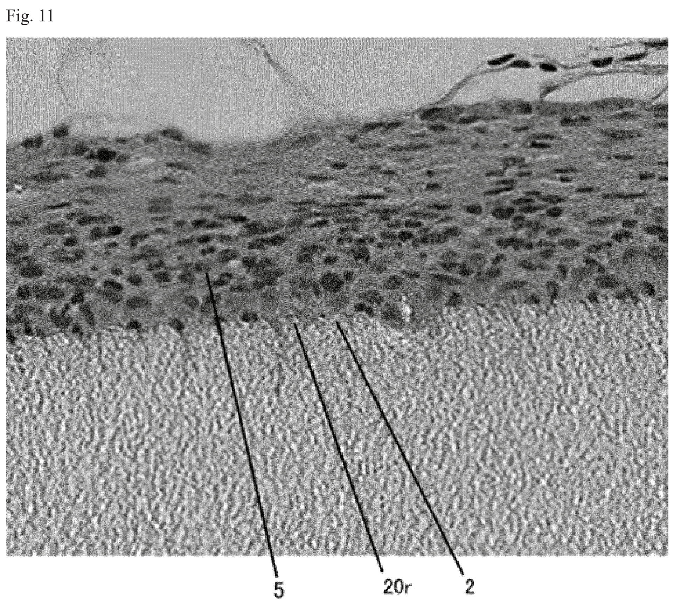

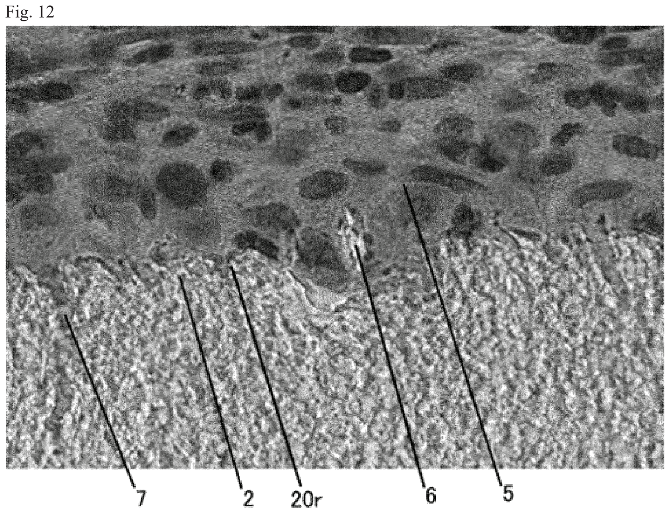

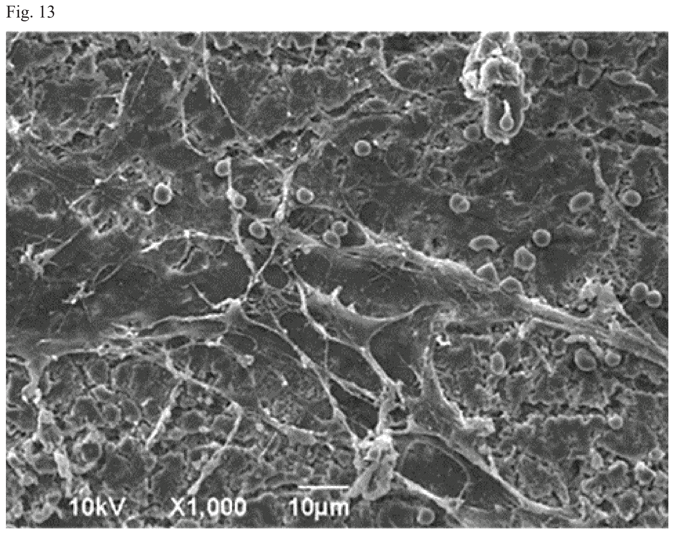

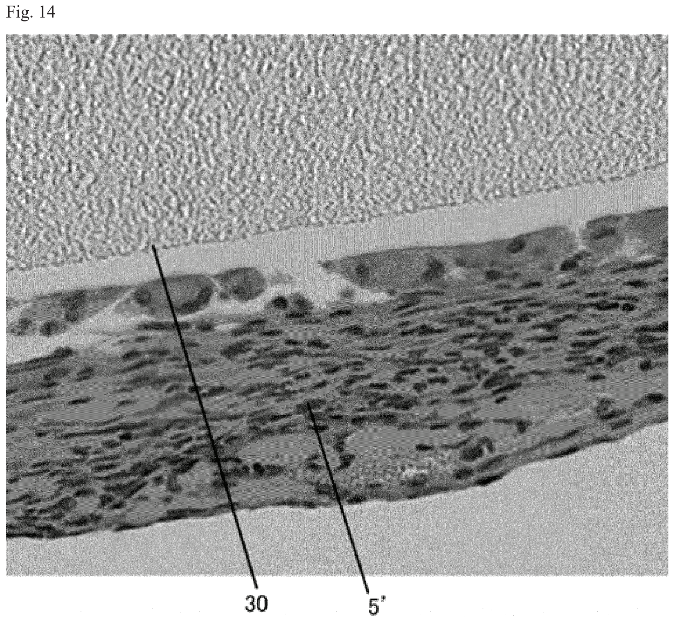

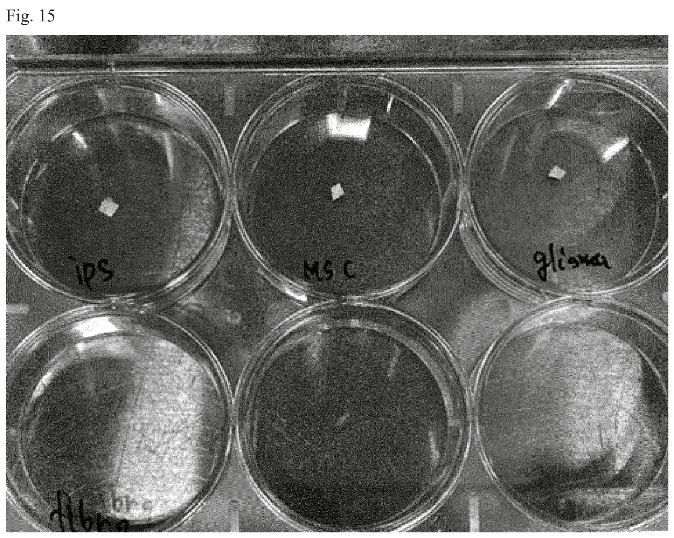

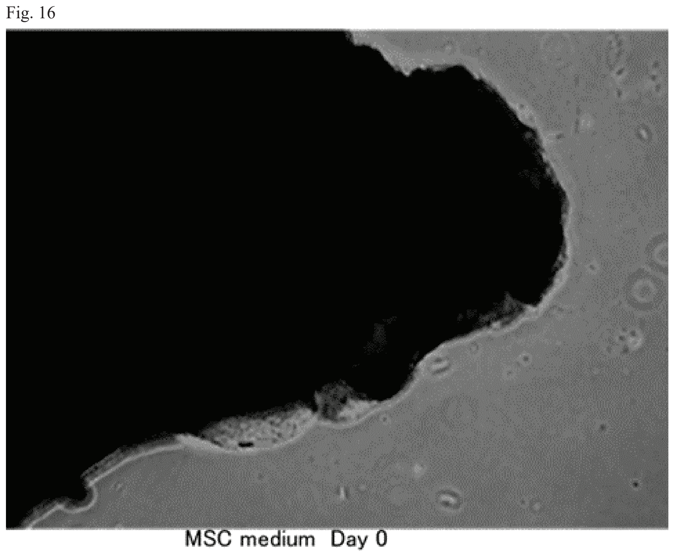

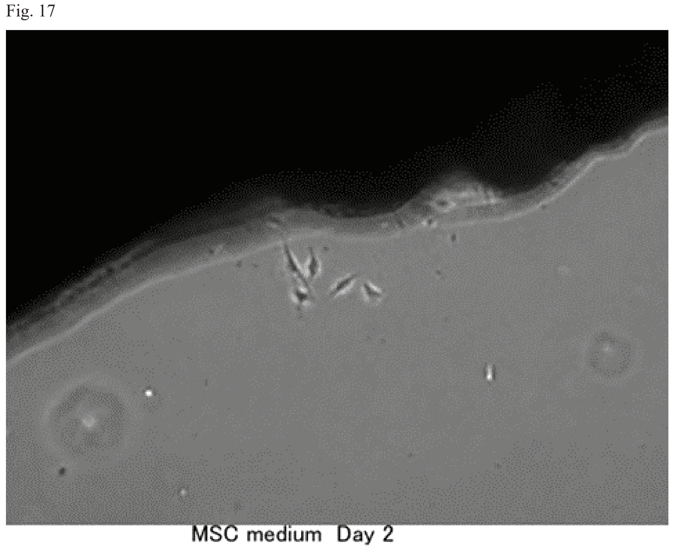

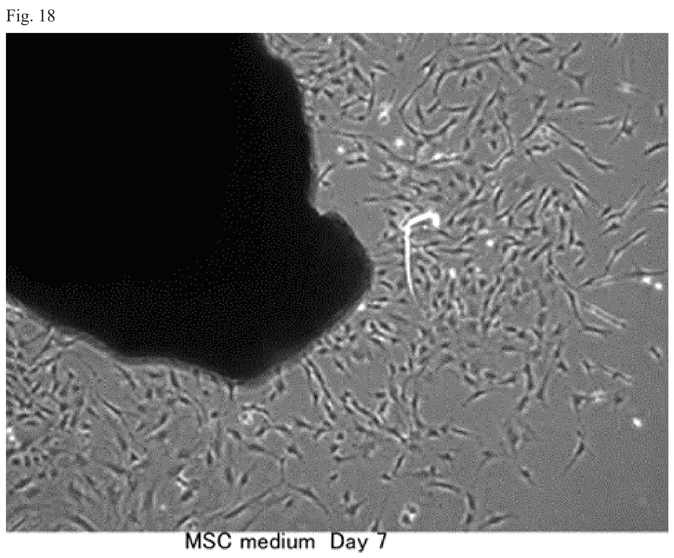

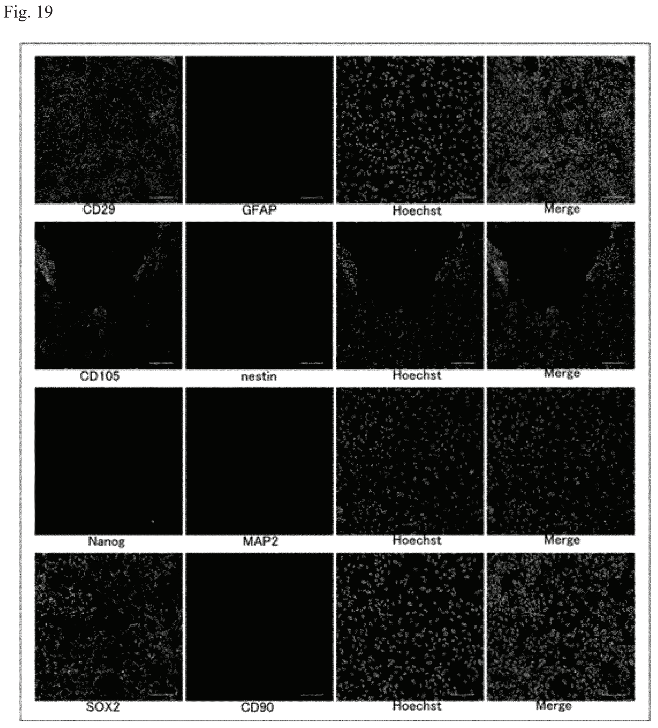

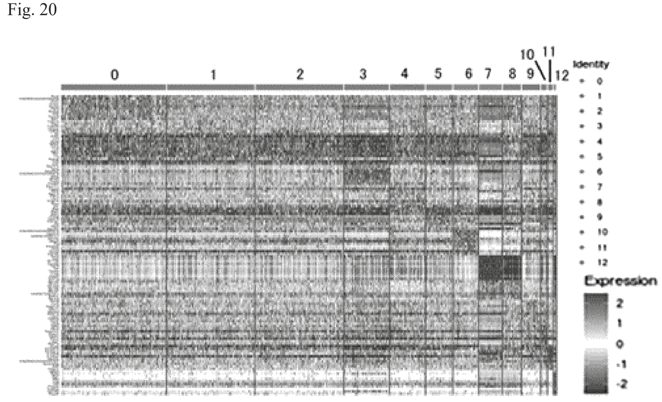

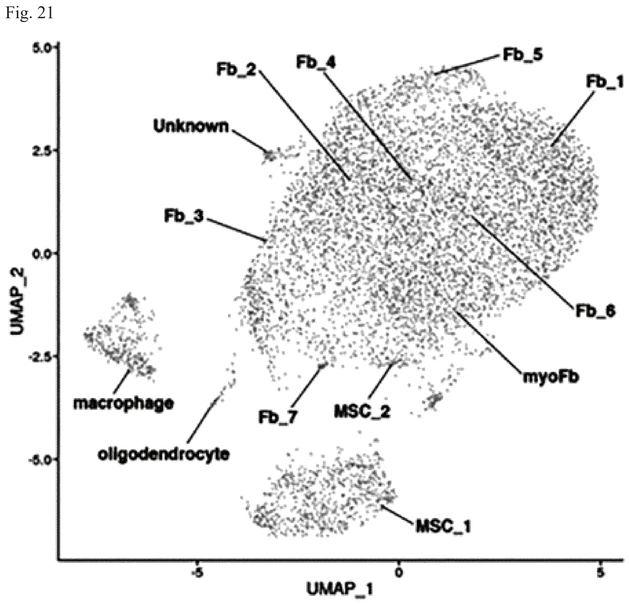

FIG. 11 is a photograph substituting for a drawing showing the state in which a biological membrane 5 was newly formed so as to interlock with the roughened surface 20r of the biological tissue regenerative treatment sheet 1A. FIG. 12 is a photograph substituting for a drawing showing a phagocytic image 6 of ePTFE. FIG. 13 is an electron micrograph of the roughened surface side of the biological tissue regenerative treatment sheet 1A. FIG. 14 is a photograph substituting for a drawing showing the state in which the second surface 30 of the biological tissue regenerative treatment sheet 1A and the biological membrane 5' are separated. FIG. 15 is a photograph substituting for a drawing showing a culture target. FIG. 16 is a micrograph showing the state of cells cultured in MSC medium after 0 days. FIG. 17 is a micrograph showing the state of cells cultured in MSC medium after 2 days. FIG. 18 is a micrograph showing the state of cells cultured in MSC medium after 7 days. FIG. 19 is a micrograph about staining of each marker. FIGS. 20 and 21 are photographs substituting for drawings showing the results of scRNA-seq analysis.

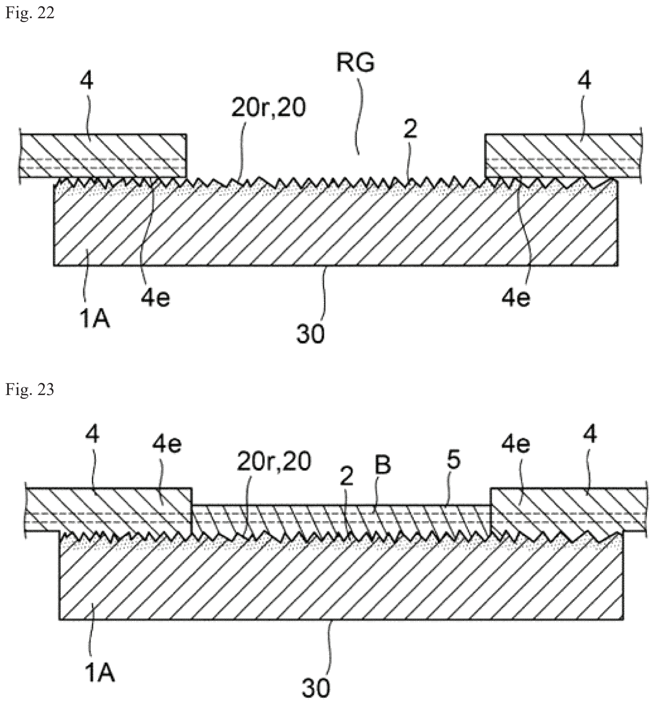

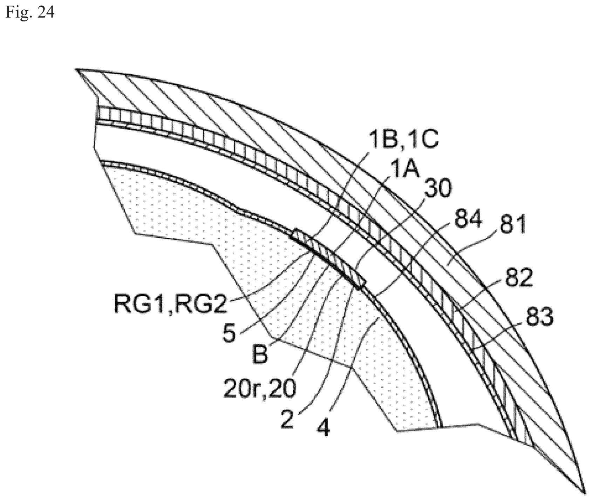

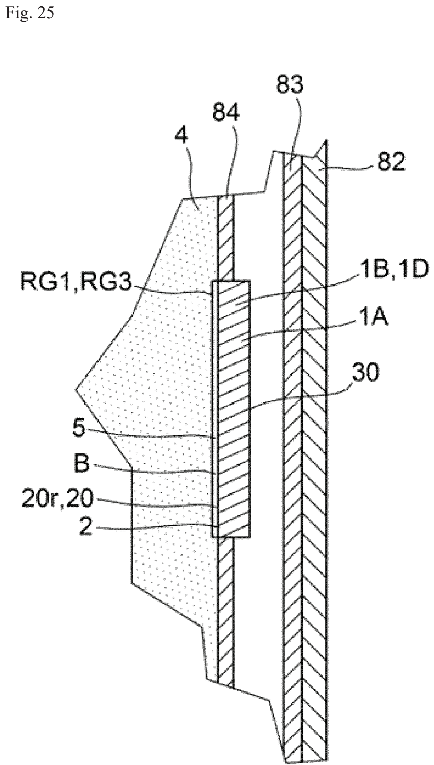

FIG. 22 is a schematic cross-sectional view schematically showing the state in which the biological tissue regenerative treatment sheet 1A according to the first embodiment was placed in in vivo tissue 4. FIG. 23 is a schematic cross-sectional view schematically showing the state in which a biological membrane 5 was newly formed along the roughened surface 20r of the biological tissue regenerative treatment sheet 1A according to the first embodiment. FIG. 24 is a schematic cross-sectional view schematically showing the state in which the biological tissue regenerative treatment sheet 1A according to the first embodiment was placed on a brain damage site. FIG. 25 is a schematic cross-sectional view schematically showing the state in which the biological tissue regenerative treatment sheet 1A according to the first embodiment was placed on a spinal cord damage site.

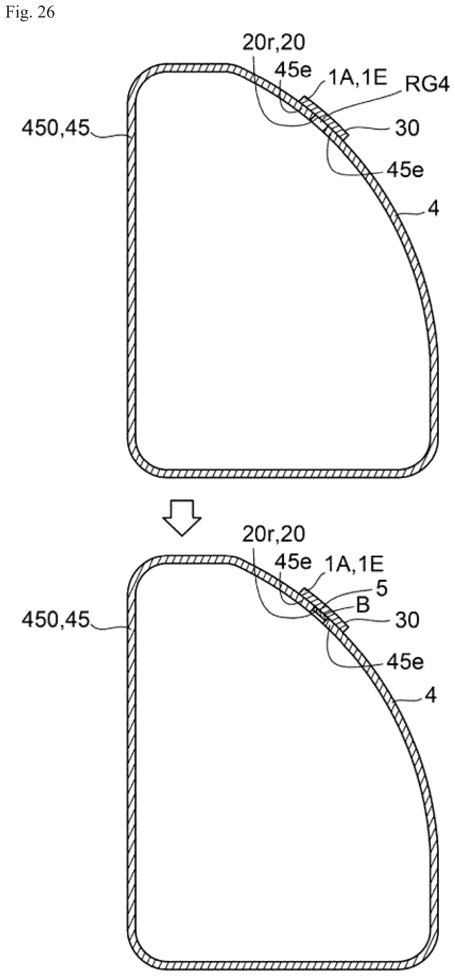

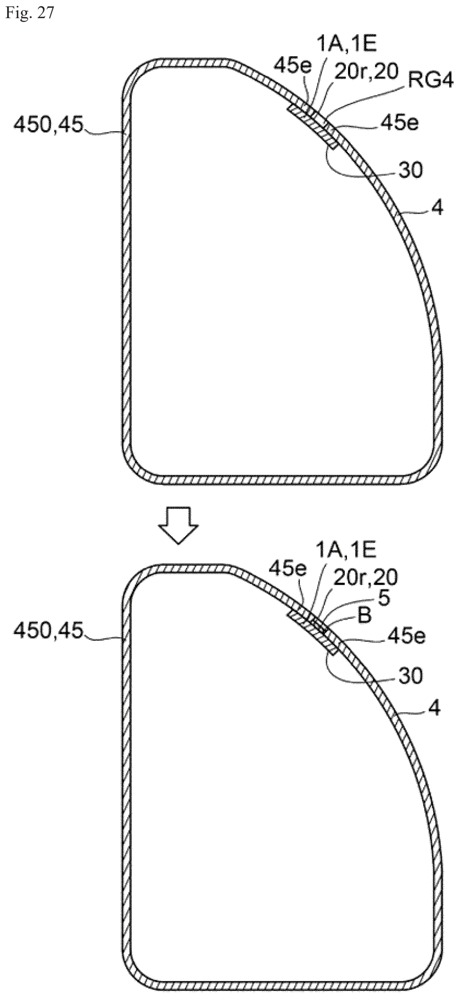

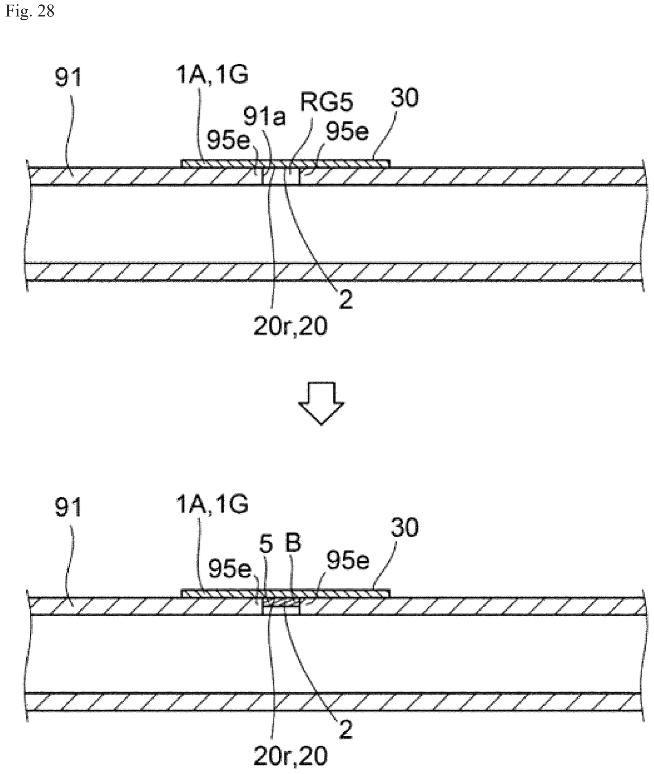

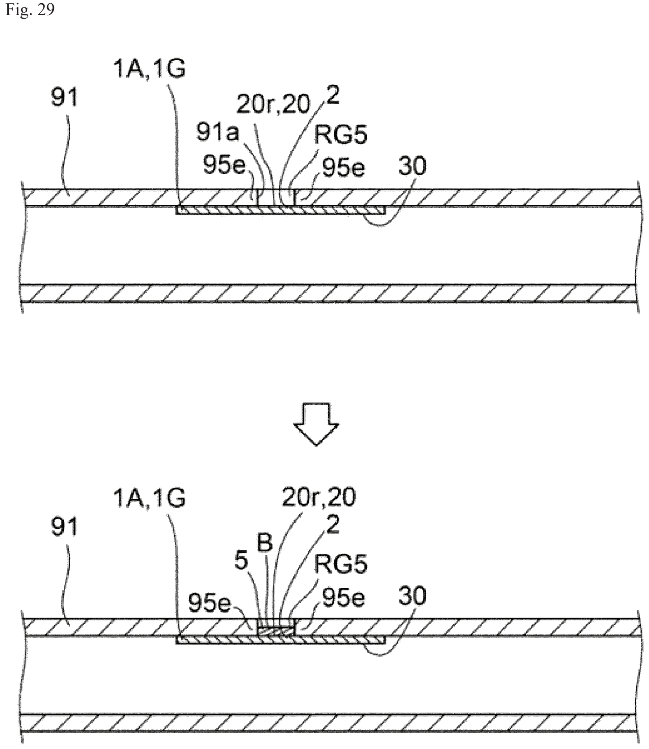

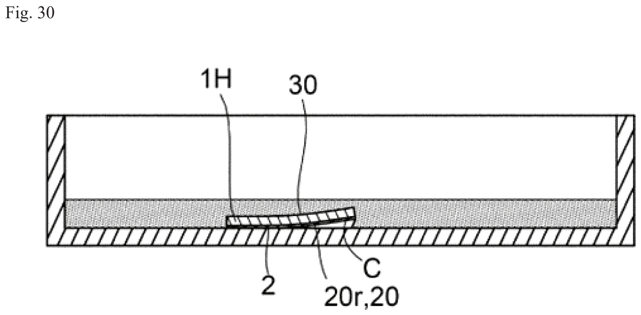

FIGS. 26 and 27 are schematic cross-sectional views schematically showing the state in which the biological tissue regenerative treatment sheet 1A according to the first embodiment was placed at a damaged site of a lung structure 45. FIGS. 28 and 29 are schematic cross-sectional views schematically showing the state in which the biological tissue regenerative treatment sheet 1A according to the first embodiment was placed on a damaged site of a blood vessel 91. FIG. 30 is a schematic cross-sectional view schematically showing the state in which cells C are cultured using a culture sheet 1H.

As illustrated in FIG. 1, the biological tissue regenerative treatment sheet 1A according to the first embodiment includes a first surface 20 and a second surface 30 disposed opposite to the first surface 20. In other words, the biological tissue regenerative treatment sheet 1A has two main surfaces, one of which is the first surface 20, and the other of which is the second surface 30.

As illustrated in FIG. 2, the first surface 20 includes a roughened surface 20r. In the example shown in FIG. 1, a part of the first surface 20 is the roughened surface 20r, and another part of the first surface 20 is a non-roughened surface 20s. Alternatively, the entire first surface 20 may be the roughened surface 20r. In this specification, "roughened surface" means a surface that has been subjected to roughening treatment. The maximum height roughness Rz of the roughened surface 20r is, for example, 8 µm or more. The "maximum height roughness Rz" is measured based on JIS B 0601:2013 (corresponding international standards ISO 4287:1997, Amd. 1:2009).

The roughened surface 20r contains polytetrafluoroethylene as a main component. In other words, the ratio of polytetrafluoroethylene constituting the roughened surface to the entire material constituting the roughened surface 20r is 50% by weight or more. The ratio of polytetrafluoroethylene constituting the roughened surface to the entire material constituting the roughened surface 20r may be 70% by weight or more, 90% by weight or more, 95% by weight or more, or 99% by weight or more.

The entire biological tissue regenerative treatment sheet 1A may mainly contain polytetrafluoroethylene. In other words, the ratio of polytetrafluoroethylene to the entire material constituting the biological tissue regenerative treatment sheet 1A may be 50% by weight or more. The ratio of polytetrafluoroethylene constituting the biological tissue regenerative treatment sheet 1A to the entire material constituting the biological tissue regenerative treatment sheet 1A may be 70% by weight or more, 90% by weight or more, 95% by weight or more, or 99% by weight or more. Alternatively, the biological tissue regenerative treatment sheet 1A may be a laminate of a layer made of polytetrafluoroethylene and a layer made of another material. In this case, at least the main component of the material constituting the roughened surface 20r should be polytetrafluoroethylene.

The second surface 30 may be a roughened surface or a smooth surface. In this specification, "smooth surface" means a surface that has not been subjected to roughening treatment and has a maximum height roughness Rz smaller than that of the roughened surface 20r.

The biological tissue regenerative treatment sheet 1A according to the first embodiment has the property that roughened polytetrafluoroethylene is phagocytosed by cells of in vivo tissue. Normally, even if polytetrafluoroethylene is brought into contact with in vivo tissue, the polytetrafluoroethylene is not phagocytosed by the cells of the in vivo tissue. In contrast, the first embodiment is configured such that the roughened surface 20r mainly containing polytetrafluoroethylene is phagocytosed by the cells of the in vivo tissue. Furthermore, regeneration of biological tissue progresses using the roughened surface 20r as a scaffold. "Phagocytosis" means the action of cells of in vivo tissue taking in unnecessary substances.

As described above, the biological tissue regenerative treatment sheet 1A according to the first embodiment achieves regeneration of biological tissue through a novel mechanism not known to date. Furthermore, the biological tissue regenerative treatment sheet 1A according to the first embodiment provides a new application for medical sheets using polytetrafluoroethylene, namely, the regeneration of biological tissue.

### Optional Additional Configuration

Next, optional additional configurations that can be adopted in the biological tissue regenerative treatment sheet 1A according to the first embodiment will be described.

### Ion-Implanted Layer 2

As illustrated in FIG. 3, the roughened surface 20r may be constituted by the surface of an ion-implanted layer 2. In this specification, "ion-implanted layer 2" means a layer whose physical and/or chemical properties have been modified by the implantation of ionized elements. In the example shown in FIG. 3, the ion-implanted layer 2 has a plurality of fine depressions 20d on its surface due to ion implantation. Furthermore, the ion-implanted layer 2 is a layer in which elements implanted into polytetrafluoroethylene by ion implantation are present as impurities. A part of the ion-implanted elements may release from the ion-implanted layer 2.

In the example shown in FIG. 3, the elements implanted into polytetrafluoroethylene by ion implantation are, for example, argon, neon, and the like. In other words, the ion-implanted layer 2 may be a layer in which argon coexists as an impurity in polytetrafluoroethylene, or a layer in which neon coexists as an impurity in polytetrafluoroethylene.

### Roughened Surface 20r

From the viewpoint of allowing the roughened surface 20r to be phagocytosed by cells, it is preferable that the roughened surface 20r has depressions 20d of approximately the same size as the cells that phagocytose the roughened surface 20r. In this specification, the depressions of approximately the same size as the cells that phagocytose the roughened surface 20r mean depressions having a depth of 1/2 to 2 times the diameter of the cells that phagocytose the roughened surface 20r. For example, the size of human macrophages is approximately 15 µm to 20 µm. Therefore, it is preferable that the roughened surface 20r has depressions 20d with a depth of 7.5 µm to 30 µm (e.g., depressions 20d with a depth of 7.5 µm to 15 µm, and/or depressions 20d with a depth of 15 µm to 30 µm), or depressions 20d with a depth of 10 µm to 40 µm (e.g., depressions 20d with a depth of 10 µm to 20 µm, and/or depressions 20d with a depth of 20 µm to 40 µm). In this case, cells that phagocytose the roughened surface 20r (e.g., macrophages) can infiltrate the depressions of the roughened surface 20r and phagocytose the roughened surface 20r.

### Expanded Polytetrafluoroethylene

In this specification, polytetrafluoroethylene may be expanded polytetrafluoroethylene (hereinafter referred to as "ePTFE"). ePTFE is polytetrafluoroethylene that has been subjected to an expansion treatment (more specifically, polytetrafluoroethylene expanded in a heated state). ePTFE is, for example, expanded porous polytetrafluoroethylene made using the method described in U.S. Patent Nos. 3,953,566 or 4,187,390. Alternatively, the polytetrafluoroethylene in this specification may be nonexpanded polytetrafluoroethylene.

### Adhesion between Roughened Surface 20r and Biological Tissue

Adhesion between the roughened surface 20r (more specifically, the ion-implanted layer 2) and the in vivo tissue may be performed via an adhesive such as fibrin glue, or may be performed without an adhesive. In other words, an adhesive may or may not be applied to the roughened surface 20r. From the viewpoint of filling the gap between the roughened surface 20r and the biological tissue with newly formed cells, it is preferable that no adhesive is applied to the roughened surface 20r.

### Second Surface 30

The second surface 30 may contain polytetrafluoroethylene as a main component. In other words, the ratio of polytetrafluoroethylene constituting the second surface 30 to the entire material constituting the second surface 30 may be 50% by weight or more. The ratio of polytetrafluoroethylene constituting the second surface 30 to the entire material constituting the second surface 30 may be 70% by weight or more, 90% by weight or more, 95% by weight or more, or 99% by weight or more. The second surface 30 is, for example, a non-roughened surface. When the second surface 30 is a non-roughened surface, adhesion between the second surface 30 and the biological tissue is prevented or suppressed. When the second surface 30 is a non-roughened surface, the second surface 30 is not the ion-implanted layer 2.

### Film Thickness of Biological Tissue Regenerative Treatment Sheet

The film thickness of the biological tissue regenerative treatment sheet 1A is, for example, 50 µm to 500 µm, 50 µm to 300 µm, 50 µm to 200 µm, or 50 µm to 150 µm. When the film thickness is sufficiently thin, it is easy to place the biological tissue regenerative treatment sheet 1A along the external shape of the in vivo tissue.

### Biological Tissue Regenerative Treatment Sheet Used in Experiments 1 to 3

Experiments 1 to 3 below were performed using a biological tissue regenerative treatment sheet 1A having a roughened surface 20r formed by argon ion (Ar+) implantation. In Experiments 1 to 3, the ratio of polytetrafluoroethylene constituting the roughened surface to the entire material constituting the roughened surface 20r of the biological tissue regenerative treatment sheet 1A was 99% by weight or more, and the ratio of polytetrafluoroethylene to the entire material constituting the biological tissue regenerative treatment sheet 1A was 99% by weight or more. The film thickness of the biological tissue regenerative treatment sheet 1A was 300 µm. The acceleration voltage of argon ions (Ar+) was 150 keV, and the implantation amount of argon ions (Ar+) into the biological tissue regenerative treatment sheet 1A was 1 × 10^14 ions/cm^2.

### Experiment 1

After anesthetizing rats (Wistar, 8 weeks old, male) by inhalation, a median incision of about 2 cm was made (FIG. 4), and a circular craniotomy of about 8 mm in diameter was performed in the right side region of the incision (FIG. 5), and the trimmed biological tissue regenerative treatment sheet 1A was placed so as to cover the craniotomy site (FIG. 6). The biological tissue regenerative treatment sheet 1A was placed so that the second surface 30 (more specifically, the non-roughened surface) faced the inside of the head, and the roughened surface 20r faced the outside of the head. Thereafter, the roughened surface 20r was covered with autologous bone and bone wax (FIG. 7), and the incision was closed.

In rats sacrificed 4.5 months after placement, the biological tissue regenerative treatment sheet 1A had become semi-transparent, and a pale yellow transparent biological membrane had formed around the biological tissue regenerative treatment sheet 1A (FIG. 8).

It was unexpected that a pale yellow transparent biological membrane would form along the roughened surface 20r facing the outside of the head. The semi-transparency of the biological tissue regenerative treatment sheet 1A was also an unexpected result (i.e., it was unforeseen that ePTFE, which is an opaque material, would become semi-transparent after being placed in the body).

### Experiment 2

After anesthetizing mice (C57BL, 8 weeks old, male) by inhalation, an opening 40a was formed in the peritoneum 40, and a biological tissue regenerative treatment sheet 1A of about 1 cm square was placed so as to close the opening 40a (FIG. 9). The biological tissue regenerative treatment sheet 1A was placed such that the roughened surface 20r was in contact with the back surface of the peritoneum 40, and the second surface 30 (more specifically, the non-roughened surface) faced the center of the abdominal cavity. The biological tissue regenerative treatment sheet 1A and the peritoneum 40 were sutured and fixed at two locations, and then the incision was closed. Mice were sacrificed 6 weeks after placement, and histological examination was performed. A semi-transparent biological membrane 5 was formed on the surface of the biological tissue regenerative treatment sheet 1A on the side of the roughened surface 20r, and clear angiogenesis of capillaries B was observed inside the biological membrane 5 (FIG. 10). Hematoxylin and eosin staining and immunohistochemical staining (FIG. 11) showed numerous multinucleated giant cells within the newly formed biological membrane 5 on the roughened surface 20r side of the biological tissue regenerative treatment sheet 1A (in other words, on the ion-implanted layer 2 side), and in some areas, phagocytic images 6 of ePTFE were also observed (FIG. 12). As can be seen from FIG. 11, new tissue (more specifically, the newly formed biological membrane 5) had developed along the roughened surface 20r of the biological tissue regenerative treatment sheet 1A, and the new tissue (more specifically, the newly formed biological membrane 5) was formed so as to interlock with the irregularities of the roughened surface 20r. Furthermore, continuous cell infiltration 7 (more specifically, cell infiltration into the interior of the ion-implanted layer 2) from the roughened surface 20r to the interior of the biological tissue regenerative treatment sheet 1A was observed (FIG. 12). When the roughened surface side of the biological tissue regenerative treatment sheet 1A (in other words, the ion-implanted layer side) was observed using a scanning electron microscope, many fibroblast-like cells and macrophage-like cells were observed on the surface of the biological tissue regenerative treatment sheet 1A (FIG. 13). On the other hand, regarding the biological membrane 5' formed on the second surface 30 (more specifically, the non-roughened surface) side of the biological tissue regenerative treatment sheet 1A, cell infiltration into the interior of the biological tissue regenerative treatment sheet 1A, ePTFE phagocytic images 6, or the like were not observed (FIG. 14).

### Experiment 3: Culture of Biological Membrane

The biological tissue regenerative treatment sheet 1A and the biological membrane formed in a state of being bonded to the biological tissue regenerative treatment sheet 1A were removed from rats, fragmented, and the biological membrane side was attached to a 6-well plate (FIG. 15). iPS cell medium (Stemflex medium (Thermo Fisher) + penicillin streptomycin + Zell shield (Funakoshi): upper left in FIG. 15), mesenchymal stem cell (hereinafter referred to as "MSC") medium (low glucose DMEM + hyclone FBS + kanamycin: upper center in FIG. 15), glioma cell medium (high glucose DMEM + Corning FBS + penicillin streptomycin: upper right in FIG. 15), and fibroblast medium (low glucose DMEM + penicillin streptomycin: lower left in FIG. 15) were added to these wells, respectively, and primary culture was performed. For the fibroblast medium, a sample in which the biological tissue regenerative treatment sheet 1A was treated with Trypsin (lower center in FIG. 15) and a sample in which only the biological membrane was attached (lower right in FIG. 15) were also cultured. Among these, cell proliferation was observed only in the MSC medium (FIG. 16: after 0 days, FIG. 17: after 2 days, FIG. 18: after 7 days). Immunostaining of cells was performed to estimate the proliferating cell type.

### Immunostaining of cells

Since cell proliferation was observed in the MSC medium, staining was performed mainly on MSC markers (FIG. 19). MSC markers CD29 and CD105 were positive, but MSC marker CD90 was negative. While neural stem cell marker SOX2 was positive, neural stem cell marker nestin was negative. Also, astrocyte marker GFAP and neuron marker MAP2 were negative. None of the markers stained all cells, suggesting that multiple cell types were coexisted. Therefore, single-cell RNA sequencing (scRNA-seq) analysis was performed to identify cell types.

### scRNA-seq analysis

scRNA-seq analysis was performed to identify the types of cells that proliferated from the biological membrane generated in conjunction with the biological tissue regenerative treatment sheet 1A. More specifically, after the biological tissue regenerative treatment sheet 1A was left in rats for 8 months, the biological tissue regenerative treatment sheet 1A and the biological membrane generated in conjunction with the biological tissue regenerative treatment sheet 1A were removed from the rats, cultured in MSC medium, and the cell suspension was submitted as a sample to a company that performs scRNA-seq. As a result of analyzing the genes expressed in each cell, it was possible to divide them into 12 cell populations (clusters) based on gene expression levels (FIG. 20). From the characteristic expression genes in each cluster, it was estimated that the proliferating cell types included fibroblasts, MSCs, macrophages, myofibroblasts, and oligodendrocytes (FIG. 21).

### Summary of experimental results and analysis results

From the results of Experiments 1 and 2, the formation of a biological membrane with angiogenesis was confirmed on the roughened surface 20r side of the biological tissue regenerative treatment sheet 1A (more specifically, on the ion-implanted layer 2 side). In addition, from the results of Experiments 1 and 2, the transparency of the biological tissue regenerative treatment sheet 1A was confirmed.

As a result of Experiment 2, when the biological tissue regenerative treatment sheet 1A was placed in the peritoneum of mice for a long period, the appearance of multinucleated cells was observed on the roughened surface 20r side (more specifically, on the ion-implanted layer 2 side). In addition, as a result of Experiment 2, cell infiltration into the interior of the ion-implanted layer 2 and phagocytic images of polytetrafluoroethylene constituting the ion-implanted layer 2 were observed.

From the results of Experiment 3, cell proliferation was observed in the culture performed using the biological tissue regenerative treatment sheet 1A and MSC medium. Furthermore, positive stem cell markers (in other words, stem cell expression) were observed by immunostaining of cells. From the results of single-cell RNA sequencing analysis, it was confirmed that the cultured cells contained fibroblasts, MSCs, macrophages, myofibroblasts, and oligodendrocytes.

From these results, it was confirmed that the biological tissue regenerative treatment sheet 1A has biocompatibility, and it was suggested that tissue repair may occur using the biological tissue regenerative treatment sheet 1A as a scaffold (in other words, the biological tissue regenerative treatment sheet 1A used in the experiment has the potential as a medical device for regenerative medicine).

### Characteristic 1 of biological tissue regenerative treatment sheet 1A

From the results of Experiment 3 and the scRNA-seq analysis, it can be said that the biological tissue regenerative treatment sheet 1A has "Characteristic 1," which is that "when the roughened surface 20r is placed in contact with damaged in vivo tissue, the expression of at least one of mesenchymal stem cells, fibroblasts, myofibroblasts, macrophages, and oligodendrocytes on the surface of the biological tissue regenerative treatment sheet is more prominent compared to when the second surface 30 is placed in contact with the damaged in vivo tissue."

Furthermore, from the results of Experiment 3 and the scRNA-seq analysis, it can be said that the biological tissue regenerative treatment sheet 1A has the characteristic that "when the roughened surface 20r is placed in contact with damaged in vivo tissue, the expression of mesenchymal stem cells on the surface of the biological tissue regenerative treatment sheet is more prominent compared to when the second surface 30 is placed in contact with the damaged in vivo tissue."

Since the biological tissue regenerative treatment sheet 1A has Characteristic 1, it can be said that this biological tissue regenerative treatment sheet 1A is useful as a medical device for regenerative medicine.

### Characteristic 2 of biological tissue regenerative treatment sheet 1A

From the results of Experiment 2 (see FIG. 10), it can be said that the biological tissue regenerative treatment sheet 1A has "Characteristic 2," which is that "when the roughened surface 20r is placed in contact with damaged in vivo tissue, capillaries B are newly formed along the roughened surface 20r."

### Characteristic 3 of Biological Tissue Regenerative Treatment Sheet 1A

As illustrated in FIGS. 22, the biological tissue regenerative treatment sheet 1A has "Characteristic 3," which is that "when the roughened surface 20r is placed in contact with the edge 4e of the in vivo tissue 4 such that the roughened surface 20r faces the defective region RG of the in vivo tissue 4, a biological membrane 5 and capillaries B are newly formed along the roughened surface 20r in the defective region RG" (see FIG. 23). This Characteristic 3 is based on events discovered in Experiment 2.

Since the biological tissue regenerative treatment sheet 1A has Characteristic 2 or Characteristic 3, it can be said that this biological tissue regenerative treatment sheet 1A is useful as a medical device for regenerative medicine. When the biological tissue regenerative treatment sheet 1A has Characteristic 2, nutrients and the like are supplied to the newly formed biological membrane 5 via the capillaries B. Therefore, the biological membrane 5 is rapidly newly formed. Furthermore, when the biological tissue regenerative treatment sheet 1A has Characteristic 3, the defective region RG of the in vivo tissue 4 is rapidly closed by the biological membrane 5.

As illustrated in FIGS. 22 and 23, the biological tissue regenerative treatment sheet 1A may have the characteristic that "when the roughened surface 20r is placed in contact with the in vivo tissue 4, new tissue develops from the in vivo tissue 4 along the roughened surface 20r, and the new tissue develops so as to interlock with the irregularities of the roughened surface 20r."

### Characteristic 4 of biological tissue regenerative treatment sheet 1A

As illustrated in FIGS. 22 and 23, the biological tissue regenerative treatment sheet 1A may have "Characteristic 4," which is that "when the roughened surface 20r is placed in contact with the in vivo tissue 4, substantially all of the gap between the in vivo tissue 4 and the roughened surface 20r is filled with new tissue." When the biological tissue regenerative treatment sheet 1A has Characteristic 4, the in vivo tissue 4 and the roughened surface 20r are firmly bonded. Furthermore, bodily fluids and the like are less likely to leak from the gap between the in vivo tissue 4 and the roughened surface 20r.

### Characteristic 5 of biological tissue regenerative treatment sheet 1A

From the results of Experiment 2 (see FIG. 12), it can be said that the biological tissue regenerative treatment sheet 1A has "Characteristic 5," which is that "cells of biological tissue infiltrate the interior of the ion-implanted layer 2."

As illustrated in FIG. 12, the biological tissue regenerative treatment sheet 1A may be configured such that cells of in vivo tissue infiltrate the depressions of the roughened surface 20r. Furthermore, the biological tissue regenerative treatment sheet 1A may be configured such that cells infiltrate the interior of a structure composed of polytetrafluoroethylene and ion-implanted elements.

### Characteristic 6 of biological tissue regenerative treatment sheet 1A

From the results of Experiment 2 (e.g., see FIG. 8), it can be said that the biological tissue regenerative treatment sheet 1A has "Characteristic 6," which is that "when the roughened surface 20r is placed in contact with in vivo tissue (e.g., in vivo membrane tissue, etc.) for 6 months or more, the opaque polytetrafluoroethylene constituting the roughened surface 20r of the biological tissue regenerative treatment sheet 1A changes from an opaque state to a transparent or semi-transparent state."

The mechanism of polytetrafluoroethylene becoming transparent or semi-transparent is unknown. However, it is inferred that one of the in vivo tissue 4, the newly formed biological membrane 5, and the cells constituting them is involved in the transparency or semi-transparency of polytetrafluoroethylene.

When polytetrafluoroethylene becomes transparent or semi-transparent, it may be possible to ascertain the state of the biological tissue on the back side of the biological tissue regenerative treatment sheet 1A.

### Nerve Damage Repair Sheet 1B

The biological tissue regenerative treatment sheet 1A according to the first embodiment described above can be used as a nerve damage repair sheet 1B. In other words, in the description of the biological tissue regenerative treatment sheet 1A according to the first embodiment, "biological tissue regenerative treatment sheet 1A" can be read as "nerve damage repair sheet 1B."

As illustrated in FIG. 24 or FIG. 25, the nerve damage repair sheet 1B includes (1) a first surface 20 including a roughened surface 20r that will be phagocytosed by cells of in vivo tissue, and (2) a second surface 30 disposed opposite to the first surface 20. The roughened surface 20r contains polytetrafluoroethylene as a main component. Since the roughened surface 20r, the first surface 20, and the second surface 30 have already been described, a repetitive description of these configurations is omitted.

The nerve damage repair sheet 1B having the characteristics 1 to 6 described above functions as a scaffold for tissue regeneration at a nerve damage site. For example, as illustrated in FIG. 24, when the roughened surface 20r is placed facing the nerve damage site RG1 of the in vivo tissue 4, a biological membrane 5 and capillaries B are newly formed along the roughened surface 20r facing the nerve damage site RG1.

The nerve damage repair sheet 1B may be configured such that "nerve cells are regenerated using the roughened surface 20r as a scaffold when the roughened surface 20r is placed to face the nerve damage site RG1 of the in vivo tissue 4".

### Brain Damage Repair Sheet 1C

The nerve damage repair sheet 1B according to the first embodiment described above can be used as a brain damage repair sheet 1C by applying it to a brain damage site. In other words, in the description of the biological tissue regenerative treatment sheet 1A (or nerve damage repair sheet 1B) according to the first embodiment, "biological tissue regenerative treatment sheet 1A" (or "nerve damage repair sheet 1B") can be read as "brain damage repair sheet 1C."

The brain damage repair sheet 1C having the characteristics 1 to 6 described above functions as a scaffold for tissue regeneration at a brain damage site. For example, as illustrated in FIG. 24, when the roughened surface 20r is placed facing the brain damage site RG2 of the in vivo tissue 4, a biological membrane 5 and capillaries B are newly formed along the roughened surface 20r facing the brain damage site RG2. In FIG. 24, the brain damage repair sheet 1C is placed in contact with the brain damage site RG2 within all of the skull 81, dura mater 82, and arachnoid mater 83.

The brain damage repair sheet 1C may be placed inside the pia mater 84. The brain damage repair sheet 1C may be attached to the pia mater 84 so as to be supported by the pia mater 84.

### Spinal Cord Damage Repair Sheet 1D

The nerve damage repair sheet 1B according to the first embodiment described above can be used as a spinal cord damage repair sheet 1D by applying it to a spinal cord damage site. In other words, in the description of the biological tissue regenerative treatment sheet 1A (or nerve damage repair sheet 1B) according to the first embodiment, "biological tissue regenerative treatment sheet 1A" (or "nerve damage repair sheet 1B") can be read as "spinal cord damage repair sheet 1D."

The spinal cord damage repair sheet 1D having the characteristics 1 to 6 described above functions as a scaffold for tissue regeneration at a spinal cord damage site. For example, as illustrated in FIG. 25, when the roughened surface 20r is placed facing the spinal cord damage site RG3 of the in vivo tissue 4, a biological membrane 5 and capillaries B are newly formed along the roughened surface 20r facing the spinal cord damage site RG3. In FIG. 25, the spinal cord damage repair sheet 1D is placed in contact with the spinal cord damage site RG3 within the dura mater 82 and the arachnoid mater 83.

The spinal cord damage repair sheet 1D may be placed inside the pia mater 84. The spinal cord damage repair sheet 1D may be attached to the pia mater 84 so as to be supported by the pia mater 84.

### Lung Damage Repair Sheet 1E

The biological tissue regenerative treatment sheet 1A according to the first embodiment described above can be used as a lung damage repair sheet 1E. In other words, in the description of the biological tissue regenerative treatment sheet 1A according to the first embodiment, "biological tissue regenerative treatment sheet 1A" can be read as "lung damage repair sheet 1E."

As illustrated in FIG. 26 or FIG. 27, the lung damage repair sheet 1E includes (1) a first surface 20 including a roughened surface 20r that will be phagocytosed by cells of biological tissue, and (2) a second surface 30 disposed opposite to the first surface 20. The roughened surface 20r contains polytetrafluoroethylene as a main component. Since the roughened surface 20r, the first surface 20, and the second surface 30 have already been described, a repetitive description of these configurations is omitted.

The lung damage repair sheet 1E having the characteristics 1 to 6 described above functions as a scaffold for tissue regeneration at a lung damage site. For example, as illustrated in FIG. 26 or FIG. 27, when the roughened surface 20r is placed in contact with the lung structure 45 (more specifically, the edge 45e defining the defective region RG4 of the lung structure 45) such that the roughened surface 20r faces the defective region RG4 (more specifically, an opening of the lung structure 45) of the lung structure 45, a biological membrane 5 and capillaries B are newly formed along the roughened surface 20r in the defective region RG4.

In this specification, lung damage includes damage to the visceral pleura 450 and damage to the parietal pleura. In the example shown in FIG. 26, there is a defective region RG4 (more specifically, an opening) in the visceral pleura 450. Furthermore, the lung damage repair sheet 1E is attached to the visceral pleura 450 so as to cover the defective region RG4. As illustrated in FIG. 26, the lung damage repair sheet 1E may be configured to be attached to the outside of the visceral pleura 450, or as illustrated in FIG. 27, it may be configured to be attached to the inside of the visceral pleura 450. In FIG. 26 or FIG. 27, the lung damage repair sheet 1E may function as a pneumothorax treatment sheet.

When the lung damage repair sheet 1E has Characteristic 4 described above, substantially all of the gap between the in vivo tissue 4 (e.g., the visceral pleura 450) and the roughened surface 20r is filled with new tissue. Therefore, air leakage from the gap between the in vivo tissue 4 and the roughened surface 20r is prevented. Furthermore, in the example shown in FIG. 26 or FIG. 27, the biological membrane 5 and capillaries B are configured to extend across the defective region RG4 using the roughened surface 20r as a scaffold. In this case, the defective region RG4 of the lung structure 45 is closed by the biological membrane 5. Therefore, lung damage is suitably repaired.

### Peritoneal Damage Repair Sheet 1F

The biological tissue regenerative treatment sheet 1A according to the first embodiment described above can be used as a peritoneal damage repair sheet 1F. In other words, in the description of the biological tissue regenerative treatment sheet 1A according to the first embodiment, "biological tissue regenerative treatment sheet 1A" can be read as "peritoneal damage repair sheet 1F."

The peritoneal damage repair sheet 1F includes (1) a first surface 20 including a roughened surface 20r that will be phagocytosed by cells of biological tissue, and (2) a second surface 30 disposed opposite to the first surface 20. The roughened surface 20r contains polytetrafluoroethylene as a main component. Since the roughened surface 20r, the first surface 20, and the second surface 30 have already been described, a repetitive description of these configurations will be omitted.

The peritoneal damage repair sheet 1F having the characteristics 1 to 6 described above functions as a scaffold for tissue regeneration at a peritoneal damage site. For example, as illustrated in FIG. 9, when the roughened surface 20r is placed in contact with the peritoneum 40 (more specifically, the edge defining the opening 40a of the peritoneum 40) such that the roughened surface 20r faces the defective region of the peritoneum 40 (more specifically, the opening 40a), a biological membrane 5 and capillaries B are newly formed along the roughened surface 20r in the defective region.

When the peritoneal damage repair sheet 1F has Characteristic 4 described above, substantially all of the gap between the peritoneum 40 and the roughened surface 20r is filled with new tissue. Therefore, leakage of bodily fluid or gas from the gap between the peritoneum 40 and the roughened surface 20r is prevented. Furthermore, in the example shown in FIG. 10, the biological membrane 5 and capillaries B are configured to extend across the defective region (see opening 40a in FIG. 9) using the roughened surface 20r as a scaffold. In this case, the defective region of the peritoneum 40 (more specifically, the opening 40a) is closed by the biological membrane 5. Therefore, peritoneal damage is suitably repaired.

### Blood Vessel Damage Repair Sheet 1G

The biological tissue regenerative treatment sheet 1A according to the first embodiment described above can be used as a blood vessel damage repair sheet 1G. In other words, in the description of the biological tissue regenerative treatment sheet 1A according to the first embodiment, "biological tissue regenerative treatment sheet 1A" can be read as "blood vessel damage repair sheet 1G."

As illustrated in FIG. 28 or FIG. 29, the blood vessel damage repair sheet 1G includes (1) a first surface 20 including a roughened surface 20r that will be phagocytosed by cells of biological tissue, and (2) a second surface 30 disposed opposite to the first surface 20. The roughened surface 20r contains polytetrafluoroethylene as a main component. Since the roughened surface 20r, the first surface 20, and the second surface 30 have already been described, a repetitive description of these configurations will be omitted.

The blood vessel damage repair sheet 1G having the characteristics 1 to 6 described above functions as a scaffold for tissue regeneration at a blood vessel damage site. For example, as illustrated in FIG. 28 or FIG. 29, when the roughened surface 20r is placed in contact with the blood vessel 91 (more specifically, the edge 95e defining the opening 91a of the blood vessel 91) such that the roughened surface 20r faces the damaged region RG5 of the blood vessel 91 (more specifically, the opening 91a of the blood vessel 91), a biological membrane 5 and capillaries B are newly formed along the roughened surface 20r in the damaged region RG5 (more specifically, in the opening 91a of the blood vessel 91).

In the example shown in FIG. 28 or FIG. 29, the blood vessel damage repair sheet 1G is attached to the blood vessel 91 so as to cover the damaged region RG5 (more specifically, the opening 91a) of the blood vessel 91. As illustrated in FIG. 28, the blood vessel damage repair sheet 1G may be configured to be attached to the outside of the blood vessel 91, or as illustrated in FIG. 29, it may be configured to be attached to the inside of the blood vessel 91.

When the blood vessel damage repair sheet 1G has Characteristic 4 described above, substantially all of the gap between the blood vessel 91 and the roughened surface 20r is filled with new tissue. Therefore, blood leakage from the gap between the blood vessel 91 and the roughened surface 20r is prevented. Furthermore, in the example shown in FIG. 28 or FIG. 29, the biological membrane 5 and capillaries B are configured to extend across the damaged region RG5 (more specifically, the opening 91a) of the blood vessel 91 using the roughened surface 20r as a scaffold. In this case, the damaged region RG5 (more specifically, the opening 91a) of the blood vessel 91 is closed by the biological membrane 5. Therefore, blood vessel damage is suitably repaired.

### Culture Sheet 1H

The biological tissue regenerative treatment sheet 1A according to the first embodiment functions well as a scaffold for biological tissue regeneration due to its roughened surface 20r. Therefore, the biological tissue regenerative treatment sheet 1A according to the first embodiment may be used as a culture sheet 1H. In other words, in the description of the biological tissue regenerative treatment sheet 1A according to the first embodiment, "biological tissue regenerative treatment sheet 1A" can be read as "culture sheet 1H."

As illustrated in FIG. 30, the culture sheet 1H according to the first embodiment includes (1) a first surface 20 including a roughened surface 20r on which target cells C for culture or biological tissue containing the cells C are placed, and (2) a second surface 30 disposed opposite to the first surface 20. The roughened surface 20r contains polytetrafluoroethylene as a main component. The roughened surface 20r is phagocytosed by the cells C mentioned above. Since the roughened surface 20r, the first surface 20, and the second surface 30 have already been described, a repetitive description of these configurations will be omitted.

The culture sheet 1H may have the characteristic that "when target cells C for culture or biological tissue containing the cells C are placed in contact with the roughened surface 20r, the expression of at least one of mesenchymal stem cells, fibroblasts, myofibroblasts, macrophages, and oligodendrocytes on the surface of the culture sheet 1H is more prominent compared to when target cells C for culture or biological tissue containing the cells C are placed in contact with the second surface 30." The cells and the like that are expressed depend on the type of target cells C for culture.

The culture sheet 1H may have the characteristic that the cells C mentioned above infiltrate the interior of the ion-implanted layer 2. The culture sheet 1H may be configured such that the cells C mentioned above infiltrate the depressions of the roughened surface 20r. Furthermore, the culture sheet H may be configured such that the cells C mentioned above infiltrate the interior of a structure composed of polytetrafluoroethylene and ion-implanted elements.

### Cerebral Infarction Treatment Sheet 1I

The biological tissue regenerative treatment sheet 1A according to the first embodiment described above can be used as a cerebral infarction treatment sheet 1I. In other words, in the description of the biological tissue regenerative treatment sheet 1A according to the first embodiment, "biological tissue regenerative treatment sheet 1A" can be read as "cerebral infarction treatment sheet 1I."

As shown in the examples described later, the use of the cerebral infarction treatment sheet 1I has been shown in animal experiments to enhance the recovery of motor function after cerebral infarction. Therefore, it is effective as a cerebral infarction treatment sheet 1I. Furthermore, as shown in the examples described later, when neovascularization increased and the cerebral infarction improved, the cerebral infarction treatment sheet 1I became transparent. Therefore, by measuring the transparency of the cerebral infarction treatment sheet 1I, it is possible to ascertain the state of neovascularization and also predict the improvement of cerebral infarction. Therefore, this specification also provides a cerebral infarction treatment sheet 1I that has the function of analyzing or ascertaining the state of neovascularization by transparency. This specification also provides a cerebral infarction treatment sheet 1I that has the function of analyzing or ascertaining the state of improvement of cerebral infarction by transparency. For example, by embedding a sensor in the brain and analyzing the cerebral infarction treatment sheet 1I, the transparency of the cerebral infarction treatment sheet 1I can be measured in real time, and the improvement of cerebral infarction and the state of neovascularization can be estimated. In this case, it is not necessary to sense the entire area of the brain, so the state of cerebral infarction can be objectively grasped with low invasiveness.

The cerebral infarction treatment sheet 1I is used, for example, by placing the ion-irradiated surface facing the brain surface.

### Angiogenesis Promoting Sheet 1J

The biological tissue regenerative treatment sheet 1A according to the first embodiment described above can be used as an angiogenesis promoting sheet 1J. In other words, in the description of the biological tissue regenerative treatment sheet 1A according to the first embodiment, "biological tissue regenerative treatment sheet 1A" can be read as "angiogenesis promoting sheet 1J."

As described above, it has been shown that using the angiogenesis promoting sheet 1J promotes angiogenesis. Therefore, this specification discloses an angiogenesis promoting sheet 1J. Furthermore, this specification can provide a sheet for various treatments (e.g., wound healing) by promoting angiogenesis using the angiogenesis promoting sheet 1J.

### Mesenchymal Stem Cell Proliferation Sheet 1K

The biological tissue regenerative treatment sheet 1A according to the first embodiment described above can be used as a mesenchymal stem cell proliferation sheet 1K. In other words, in the description of the biological tissue regenerative treatment sheet 1A according to the first embodiment, "biological tissue regenerative treatment sheet 1A" can be read as "mesenchymal stem cell proliferation sheet 1K."

As shown in the examples described later, it was demonstrated that mesenchymal stem cells proliferated by using the mesenchymal stem cell proliferation sheet 1K. In particular, it was shown that mesenchymal stem cells proliferated when the artificial dura mater after placement of the mesenchymal stem cell proliferation sheet 1K was cultured. As described above, the sheet of the present invention functions as an angiogenesis promoting sheet. This may be because mesenchymal stem cells can differentiate into blood vessels, and the sheet of the present invention proliferates mesenchymal stem cells, which contributes to angiogenesis.

Therefore, the mesenchymal stem cell proliferation sheet 1K of the present invention is effective in treating diseases related to mesenchymal stem cells. Furthermore, when the mesenchymal stem cell proliferation sheet 1K of the present invention is cultured in vivo, components contained in the culture supernatant are produced. Therefore, the mesenchymal stem cell proliferation sheet 1K can also be effectively used as a treatment sheet for diseases where the culture supernatant functions.

### Anti-inflammatory Sheet 1L

The biological tissue regenerative treatment sheet 1A according to the first embodiment described above can be used as an anti-inflammatory sheet 1L. In other words, in the description of the biological tissue regenerative treatment sheet 1A according to the first embodiment, "biological tissue regenerative treatment sheet 1A" can be read as "anti-inflammatory sheet 1L." That is, as described above, the sheet of the present invention promotes the proliferation of mesenchymal stem cells, and along with this, mesenchymal stem cells secrete various components. Therefore, the sheet of the present invention effectively functions as an anti-inflammatory sheet 1L.

The cerebral infarction treatment sheet 1I, the angiogenesis promoting sheet 1J, the mesenchymal stem cell proliferation sheet 1K, and the anti-inflammatory sheet 1L can be used and modified in the same way as other uses of the biological tissue regenerative treatment sheet 1A.

### Second Embodiment

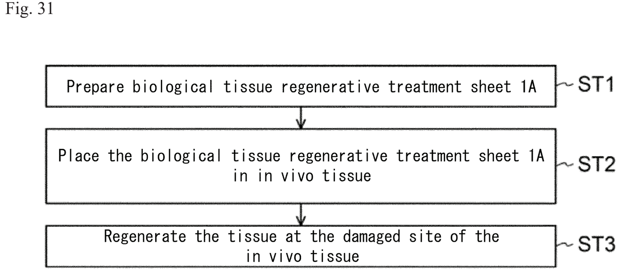

A regenerative treatment method according to the second embodiment will be described with reference to FIGS. 1 to 31. FIG. 31 is a flowchart showing an example of a regenerative treatment method according to the second embodiment.

As illustrated in FIG. 2, in the first step ST1, a biological tissue regenerative treatment sheet 1A is prepared. The first step ST1 is a preparation step.

The biological tissue regenerative treatment sheet 1A prepared in the preparation step (first step ST1) has a first surface 20 at least partially composed of a roughened surface 20r containing polytetrafluoroethylene as a main component, and a second surface 30 disposed opposite to the first surface 20.

The preparation step (first step ST1) may include forming an ion-implanted layer 2 in the biological tissue regenerative treatment sheet 1A by irradiating the first surface 20 with ions (e.g., argon ions, neon ions, etc.). By irradiating the first surface 20 with ions, the irradiated portion of the first surface 20 becomes the roughened surface 20r. Furthermore, the roughened surface 20r will be constituted by the surface of the ion-implanted layer 2.

The preparation step (first step ST1) may include applying a heat treatment to the ion-implanted biological tissue regenerative treatment sheet 1A. Ion implantation and heat treatment relax the regularity of the polymer structure of polytetrafluoroethylene. This relaxation of regularity may make it easier for polytetrafluoroethylene to become transparent or semi-transparent in the regeneration step described later. In this heat treatment, the biological tissue regenerative treatment sheet 1A may be placed in a heated atmosphere of 100 degrees Celsius or more.

Since the biological tissue regenerative treatment sheet 1A has already been described in the first embodiment, a repetitive description of the biological tissue regenerative treatment sheet 1A will be omitted.

As illustrated in FIG. 6 or FIG. 9, in the second step ST2, the biological tissue regenerative treatment sheet 1A is placed in in vivo tissue. The second step ST2 is a placement step.

In the placement step (second step ST2), the biological tissue regenerative treatment sheet 1A is placed in damaged in vivo tissue such that the roughened surface 20r is in contact with the damaged in vivo tissue. The in vivo tissue 4 in which the biological tissue regenerative treatment sheet 1A is placed is, for example, the brain, spinal cord, lung structure, peritoneum, or blood vessel. The in vivo tissue 4 in which the biological tissue regenerative treatment sheet 1A is placed may be the pia mater covering the brain, the pia mater covering the spinal cord, or the visceral pleura.

As illustrated in FIG. 22, the placement step (second step ST2) may include placing the roughened surface 20r in contact with the in vivo tissue 4 (more specifically, the edge 4e defining the defective region RG) such that the roughened surface 20r faces the defective region RG (e.g., an opening of the in vivo tissue 4) of the in vivo tissue 4.

In the third step ST3, the tissue at the damaged site of the in vivo tissue is regenerated. The third step ST3 is a regeneration step.

In the regeneration step (third step ST3), the tissue at the damaged site of the in vivo tissue 4 is regenerated using the roughened surface 20r as a scaffold. The regeneration step (third step ST3) includes the cells of the in vivo tissue 4 phagocytosing at least a portion of the roughened surface 20r. More specifically, the regeneration step (third step ST3) includes the cells of the in vivo tissue 4 phagocytosing polytetrafluoroethylene constituting the roughened surface 20r. The regeneration step (third step ST3) may include the cells of the in vivo tissue 4 phagocytosing polytetrafluoroethylene constituting the ion-implanted layer 2.

The regeneration step (third step ST3) may include newly forming a biological membrane 5 along the biological tissue regenerative treatment sheet 1A. The biological membrane 5 may include at least one of mesenchymal stem cells, fibroblasts, myofibroblasts, macrophages, and oligodendrocytes. Alternatively, or additionally, the biological membrane 5 may include capillaries B.

If the placement step (second step ST2) includes placing the roughened surface 20r to face the defective region RG of the in vivo tissue 4, the regeneration step (third step ST3) may include newly forming a biological membrane 5 and capillaries B along the roughened surface 20r in the defective region RG. The regeneration step (third step ST3) may include newly forming the biological membrane 5 such that the defective region RG is closed by the biological membrane 5.

The regeneration step (third step ST3) may include filling substantially all of the gap between the in vivo tissue 4 and the roughened surface 20r with new tissue. Furthermore, the regeneration step (third step ST3) may include infiltrating cells of in vivo tissue into the interior of the ion-implanted layer 2. The regeneration step (third step ST3) may include infiltrating cells of in vivo tissue into the depressions of the roughened surface 20r. Furthermore, the regeneration step (third step ST3) may include infiltrating cells into the interior of a structure composed of polytetrafluoroethylene and ion-implanted elements.

The regeneration step (third step ST3) may include changing the state of the polytetrafluoroethylene constituting the roughened surface 20r (more specifically, the polytetrafluoroethylene constituting the ion-implanted layer 2) from an opaque state to a transparent or semi-transparent state.

The regeneration step (third step ST3) may include repairing nerve damage by regenerating the tissue at the damaged site of the in vivo tissue 4 using the roughened surface 20r as a scaffold.

The regeneration step (third step ST3) may include repairing brain damage or spinal cord damage by regenerating the tissue at the damaged site of in vivo tissue using the roughened surface 20r as a scaffold. The regeneration step (third step ST3) may include regenerating nerve cells using the roughened surface 20r as a scaffold.

The regeneration step (third step ST3) may include repairing lung damage, peritoneal damage, or blood vessel damage by regenerating the tissue at the damaged site of in vivo tissue using the roughened surface 20r as a scaffold. The regeneration step (third step ST3) may include newly forming a biological membrane 5 in an opening of a lung structure, an opening of the peritoneum, or an opening of a blood vessel by regenerating the tissue at the damaged site of in vivo tissue using the roughened surface 20r as a scaffold.

The present invention is not limited to the above embodiments or modifications, and it is obvious that each embodiment or modification can be appropriately modified or changed within the scope of the technical concept of the present invention. Furthermore, various technologies used in each embodiment or modification can be applied to other embodiments or modifications as long as no technical contradiction arises. Furthermore, optional additional configurations in each embodiment or modification can be omitted as appropriate.

The biological tissue regenerative treatment sheet, nerve damage repair sheet, brain damage repair sheet, spinal cord damage repair sheet, lung damage repair sheet, peritoneal damage repair sheet, or blood vessel damage repair sheet in the embodiments may be applied to in vivo tissue of humans or in vivo tissue of animals other than humans.

### Examples

Hereinafter, the present invention will be specifically described using examples.

### Example 1

### Cerebral Infarction Treatment Sheet 1I

A biological tissue regenerative treatment sheet 1A having a roughened surface 20r formed by argon ion (Ar+) implantation was prepared and used as a cerebral infarction treatment sheet 1I. The ratio of polytetrafluoroethylene constituting the roughened surface to the entire material constituting the roughened surface 20r of the cerebral infarction treatment sheet 1I was 99% by weight or more, and the ratio of polytetrafluoroethylene to the entire material constituting the cerebral infarction treatment sheet 1I was 99% by weight or more. The film thickness of the cerebral infarction treatment sheet 1I was 300 µm. The acceleration voltage of argon ions (Ar+) was 150 keV, and the implantation amount of argon ions (Ar+) into the cerebral infarction treatment sheet 1I was 1 × 10^14 ions/cm^2.

Distal middle cerebral artery occlusion (Distal MCA occlusion) model mice (C57BL, 9 weeks old, male) were prepared as follows. Mice were anesthetized by inhalation, the left common carotid artery was exposed, and temporary ligation was performed with 6-0 nylon. The temporal region was incised, the temporalis muscle was peeled from the linea temporalis, the temporal bone was exposed, and the distal left middle cerebral artery visible through was confirmed. The bone directly above the middle cerebral artery was removed with a drill, the middle cerebral artery was cauterized with a bipolar, and cut with a pointed tool to create a distal middle cerebral artery occlusion (Distal MCA occlusion) model mouse.

These mice were kept on a 37°C hot plate for 1 hour, and the common carotid artery ligation was released. Thereafter, they were fixed on a stereotaxic frame and external decompression was performed with a drill. Next, the trimmed cerebral infarction treatment sheet 1I was placed so as to straddle the normal brain and the infarcted brain, and the incision was closed.

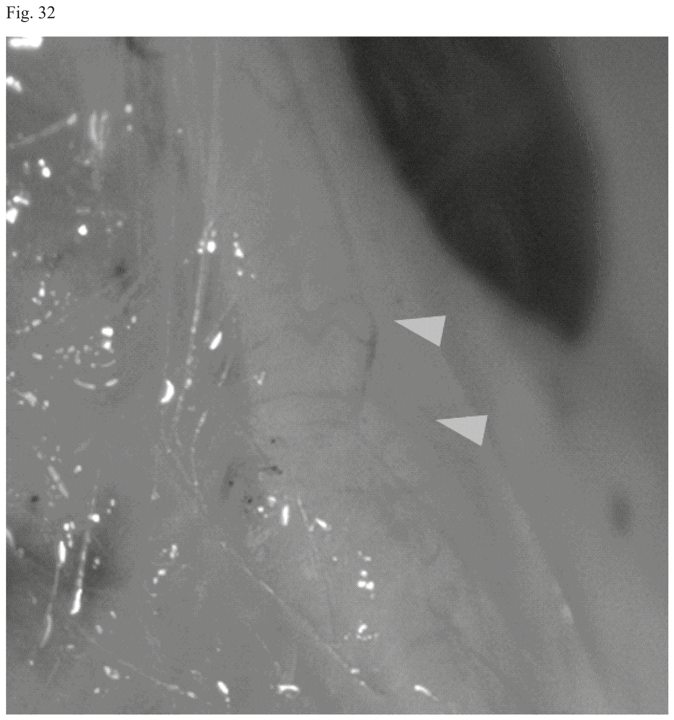

FIG. 32 shows the cerebral infarction treatment sheet 1I placed at the location indicated by the triangle. For the placement of the cerebral infarction treatment sheet 1I, two groups were established: a roughened surface inner group (n=3) in which the roughened surface was placed facing the inside of the head, i.e., in contact with the brain, and a roughened surface outer group (n=3) in which the roughened surface was placed facing the outside of the head, i.e., the non-roughened surface was in contact with the brain.

The roughened surface inner group and the roughened surface outer group were bred for 5.5 months after placement of the cerebral infarction treatment sheet 1I. Thereafter, the rotarod test and corner test were performed to investigate the recovery of motor function in the mice.

### Rotarod Test

The rotarod is a device having a rotating rod horizontally placed to evaluate an animal's motor coordination and balance. After placing the mouse on the rod, the rod starts to rotate, and the time until the mouse falls off the rod is recorded. The faster the mouse falls off the rod without being able to continue walking, the greater the decline in motor function due to cerebral infarction. Conversely, if the time until the mouse falls off the rod becomes longer, it is considered that motor function has recovered and the symptoms of cerebral infarction have improved.

In this example, a rotarod for rats and mice (MK-670 manufactured by Muromachi Kikai Co., Ltd.) was used. The rotarod was set to a mode where it accelerates to 40 rpm/60 seconds for up to 60 seconds, and then continues to rotate at 40 rpm for up to 300 seconds. The test was conducted according to the following procedure.
(1) Training was performed several times with the same mode on the day before the surgery.
(2) The test was performed on the day of surgery and just before surgery, 4 days after surgery, 7 days after surgery, 14 days after surgery, and 28 days after surgery.
(3) The time in seconds until falling off the rod was measured twice, and the average was taken. All values were divided by the value just before surgery on the day of surgery.

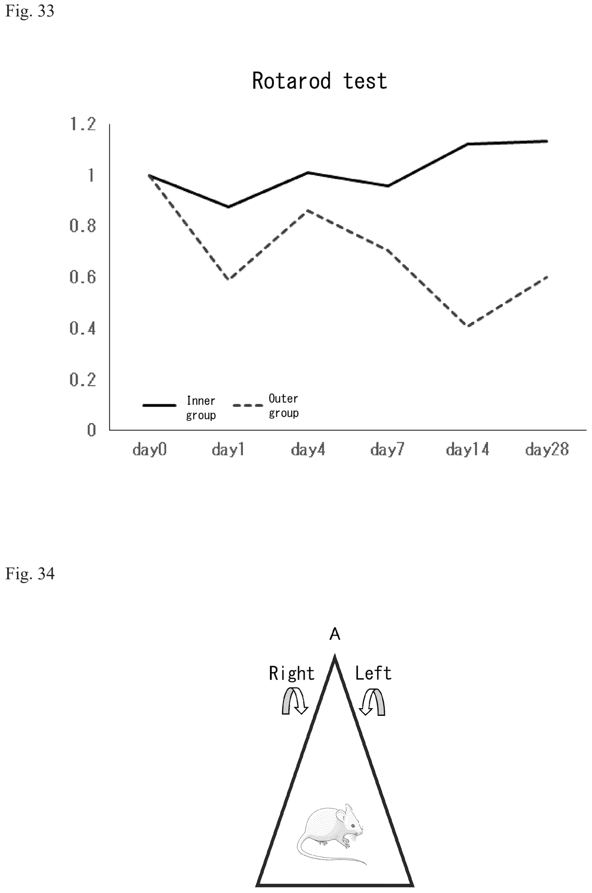

The test results are shown in FIG. 33. As can be seen from FIG. 33, the roughened surface inner group showed faster recovery of motor function compared to the roughened surface outer group. This suggests that the use of the cerebral infarction treatment sheet 1I induced the migration of mesenchymal stem cells and angiogenesis, thereby promoting nerve function recovery. Therefore, the biological tissue regenerative treatment sheet of the present invention is useful as a cerebral infarction treatment sheet.

### Corner Test

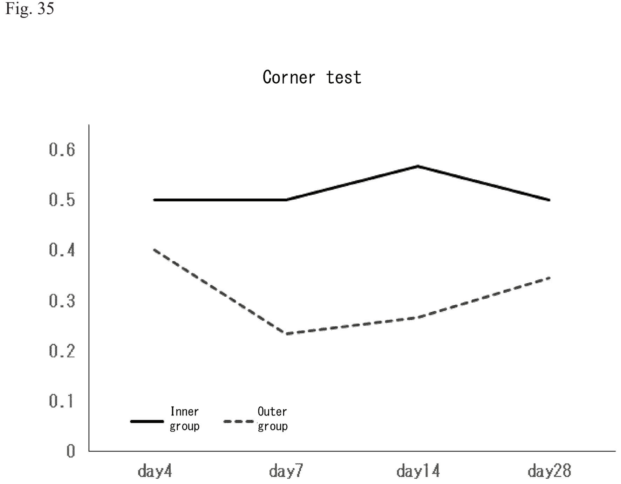

A corner (triangular prism) as shown in FIG. 34 was made of cardboard, and mice were placed inside. When the mouse went to vertex A, it was observed and recorded whether it turned right or left. The same test was performed 10 times, the number of right turns and left turns at vertex A was recorded, and the number of right turns / (number of right turns + number of left turns) was calculated. It was determined that the closer this value was to 0, the stronger the left paralysis; the closer to 1, the stronger the right paralysis; and the closer to 0.5, the weaker the paralysis. The results are shown in FIG. 35.

As can be seen from FIG. 35, the roughened surface inner group showed faster recovery of paralysis symptoms compared to the roughened surface outer group. This suggests that the use of the cerebral infarction treatment sheet 1I induced the migration of mesenchymal stem cells and angiogenesis, thereby promoting nerve function recovery. Therefore, the biological tissue regenerative treatment sheet of the present invention is useful as a cerebral infarction treatment sheet 1I.

### Angiogenesis Promoting Sheet 1J

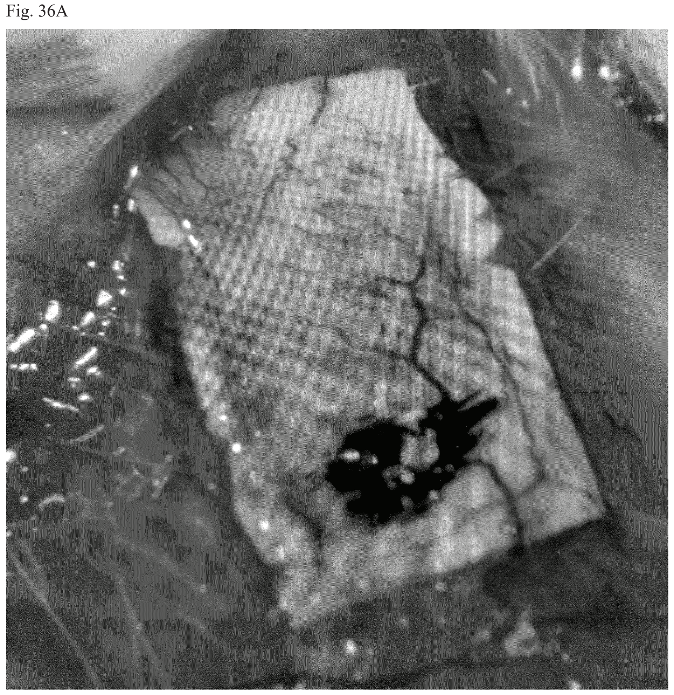

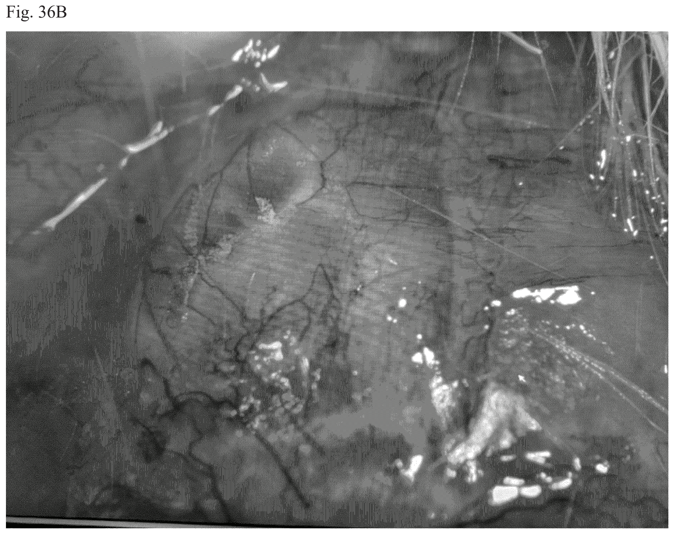

The mice in which the cerebral infarction treatment sheet 1I was placed were subjected to re-craniotomy after 5.5 months of placement, and the surface of the cerebral infarction treatment sheet 1I was observed. As shown in FIG. 36, it was observed that extracranial blood vessels extended onto or into the cerebral infarction treatment sheet 1I. In particular, more angiogenesis was observed in the roughened surface inner group in FIG. 36(b) compared to the roughened surface outer group in FIG. 36(a). This suggested that the biological tissue regenerative treatment sheet 1A has an angiogenesis promoting effect. Therefore, it was found that the biological tissue regenerative treatment sheet 1A of the present invention also functions as an angiogenesis promoting sheet 1J.

### Example 2

### Angiogenesis Promoting Sheet 1J

The same biological tissue regenerative treatment sheet 1A as in Example 1 was prepared and used as an angiogenesis promoting sheet 1J.

After anesthetizing mice (C57BL, 8 weeks old, male) by inhalation, an opening 40a was formed in the peritoneum 40, and a biological tissue regenerative treatment sheet 1A of about 1 cm square was placed so as to close the opening 40a (FIG. 9). The biological tissue regenerative treatment sheet 1A was placed such that the roughened surface 20r was in contact with the back surface of the peritoneum 40, and the second surface 30 (more specifically, the non-roughened surface) faced the center of the abdominal cavity. The biological tissue regenerative treatment sheet 1A and the peritoneum 40 were sutured and fixed at two locations, and then the incision was closed. Mice were sacrificed 6 weeks after placement, and histological examination was performed.

A semi-transparent biological membrane 5 was formed on the surface of the biological tissue regenerative treatment sheet 1A on the side of the roughened surface 20r, and clear angiogenesis of capillaries B was observed inside the biological membrane 5 (FIG. 10). That is, in Example 2, blood vessels of the abdominal wall extended into the biological tissue regenerative treatment sheet 1A. This suggested that the biological tissue regenerative treatment sheet 1A has an angiogenesis promoting effect. Therefore, it was found that the biological tissue regenerative treatment sheet 1A of the present invention also functions as an angiogenesis promoting sheet 1J.

### Example 3

### Mesenchymal Stem Cell Proliferation Sheet 1K

The same biological tissue regenerative treatment sheet 1A as in Example 1 was prepared and used as a mesenchymal stem cell proliferation sheet 1K.

After anesthetizing rats (Wistar, 8 weeks old, male) by inhalation, a median incision of about 2 cm was made (FIG. 4), and a circular craniotomy of about 8 mm in diameter was performed in the right side region of the incision (FIG. 5), and the trimmed biological tissue regenerative treatment sheet 1A was placed so as to cover the craniotomy site (FIG. 6). The biological tissue regenerative treatment sheet 1A was placed so that the second surface 30 (more specifically, the non-roughened surface) faced the inside of the head, and the roughened surface 20r faced the outside of the head. Thereafter, the roughened surface 20r was covered with autologous bone and bone wax (FIG. 7), and the incision was closed.

In rats sacrificed 4.5 months after placement, the biological tissue regenerative treatment sheet 1A had become semi-transparent, and a pale yellow transparent biological membrane had formed around the biological tissue regenerative treatment sheet 1A (FIG. 8).

The biological tissue regenerative treatment sheet 1A and, the biological membrane formed and bonded to the biological tissue regenerative treatment sheet 1A, were removed from rats, fragmented, and the biological membrane side was attached to a 6-well plate (FIG. 15). To one of these, mesenchymal stem cell (hereinafter referred to as "MSC") medium (low glucose DMEM + hyclone FBS + kanamycin: upper center in FIG. 15) was added, and primary culture was performed. Cell proliferation was observed in this well (FIG. 16: after 0 days, FIG. 17: after 2 days, FIG. 18: after 7 days).

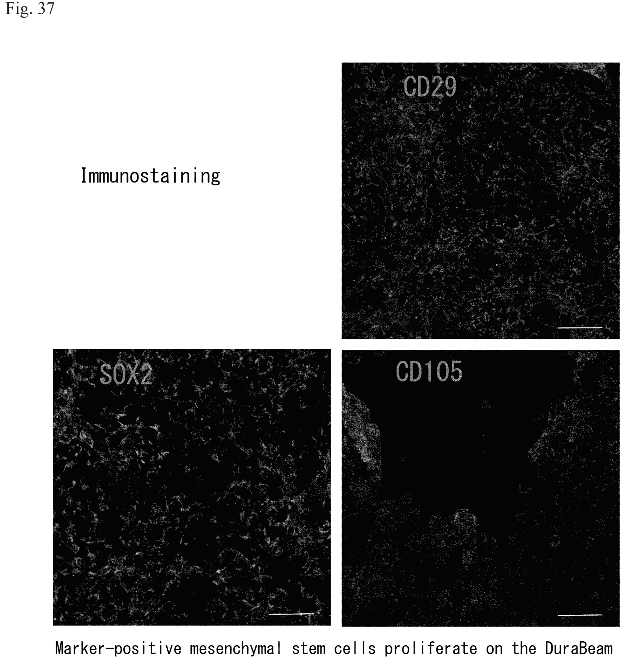

Since cell proliferation was observed in the MSC medium, staining was performed on MSC markers (FIG. 37). MSC markers CD29 and CD105 were positive. MSC marker CD90 was negative. While neural stem cell marker SOX2 was positive, neural stem cell marker nestin was negative. These results are for biological tissue regenerative treatment. These results suggest that the bio-tissue regenerative therapeutic sheet 1A has the effect of promoting the proliferation of mesenchymal stem cells. Therefore, the bio-tissue regenerative therapeutic sheet 1A of the present invention can be used as a mesenchymal stem cell proliferation sheet 1K.

### Example 4

### Anti-inflammatory Sheet 1L

In Example 4, single-cell RNA sequencing (scRNA-seq) analysis was conducted on cells proliferated on the biological tissue regenerative treatment sheet 1A.

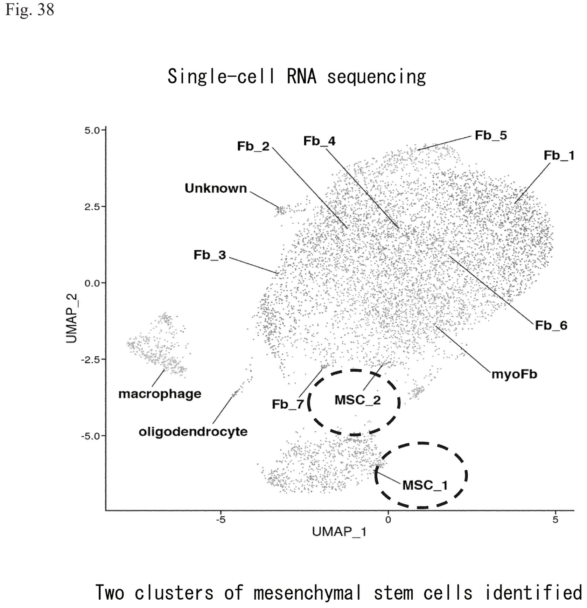

A biological tissue regenerative treatment sheet 1A of the same as Example 1 was prepared. As in Example 3, the biological tissue regenerative treatment sheet 1A was placed in rats (Wistar, 8 weeks old, male). Eight months after placement, the biological tissue regenerative treatment sheet 1A and the biological membrane formed in conjunction with the sheet 1A were retrieved from the rats. As in Example 3, the retrieved samples were cultured in medium for MSCs, and the resulting cell suspension was submitted to a company for scRNA-seq analysis. The result of the analysis of the genes expressed in each cell showed that, based on gene expression levels, the cells were classified into 12 distinct clusters (Figure 20). Among them, two clusters of mesenchymal stem cells were identified (Figure 38) This indicates that mesenchymal stem cells proliferate on the biological tissue regenerative treatment sheet 1A.

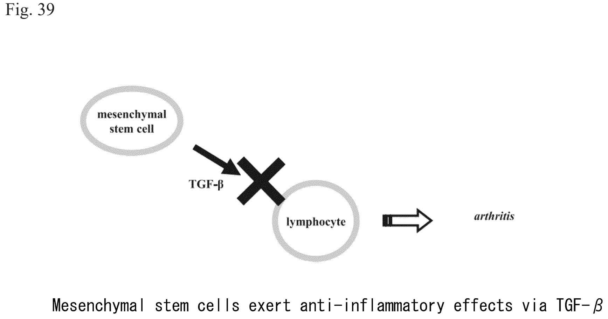

According to a report by Tanaka et al., mesenchymal stem cells exert immunosuppressive effects via TGF-β (Yoshiya Tanaka, et al. Treatment and regeneration of inflammatory arthritis using mesenchymal cells. The Journal of the Japanese Society for Immunology. 2015) (Figure 39).

Taken together, these results suggest that the biological tissue regenerative treatment sheet 1A suppresses immune responses by promoting mesenchymal stem cell proliferation, i.e., it has an anti-inflammatory effect. Therefore, the biological tissue regenerative treatment sheet 1A of the present invention is useful as an anti-inflammatory sheet 1L.

### Explanation of symbols

1A: biological tissue regenerative treatment sheet
1B: nerve damage repair sheet
1C: brain damage repair sheet
1D: spinal cord damage repair sheet
1E: lung damage repair sheet
1F: peritoneal damage repair sheet
1G: blood vessel damage repair sheet
1H: culture sheet
2: ion-implanted layer
4: in vivo tissue
4e: edge
5: biological membrane
5': biological membrane
6: phagocytic image
7: cell infiltration
20: first surface
20d: depression
20r: roughened surface
20s: non-roughened surface
30: second surface
40: peritoneum
40a: opening
45: lung structure:
45e: edge
81: skull
82: dura mater
83: arachnoid mater
84: pia mater
91: blood vessel
91a: opening
95e: edge
450: visceral pleura
B: capillaries
C: cells
H: culture sheet
RG: defective region
RG1: nerve damage site
RG2: brain damage site
RG3: spinal cord damage site
RG4: defective region RG
5: damage region

## Claims

1. A nerve damage repair sheet, comprising:
a first surface including a roughened surface to be brought into contact with a site of nerve damage and phagocytosed by in vivo tissue cells; and
a second surface disposed opposite to the first surface;
wherein
the roughened surface comprises polytetrafluoroethylene as a main component, and
wherein
the roughened surface is brought into contact with a site of nerve damage,
whereby the roughened surface is phagocytosed by in vivo tissue cells,
mesenchymal stem cells are expressed on the roughened surface,
a capillary is newly formed along the roughened surface, and
nerve cells at the site of nerve damage are regenerated using the roughened surface as a scaffold.

2. A nerve damage repair sheet, comprising:
a first surface including a roughened surface to be brought into contact with a site of nerve damage in the brain and phagocytosed by in vivo tissue cells; and
a second surface disposed opposite to the first surface;
wherein
the roughened surface comprises polytetrafluoroethylene as a main component, and
wherein
the roughened surface is brought into contact with a site of nerve damage in the brain,
whereby the roughened surface is phagocytosed by in vivo tissue cells,
mesenchymal stem cells are expressed on the roughened surface,
capillaries are newly formed along the roughened surface, and
nerve cells at the site of nerve damage in the brain are regenerated using the roughened surface as a scaffold.

3. A nerve damage repair sheet, comprising:
a first surface including a roughened surface to be brought into contact with a site of nerve damage in the spinal cord and phagocytosed by in vivo tissue cells; and
a second surface disposed opposite to the first surface;
wherein
the roughened surface comprises polytetrafluoroethylene as a main component, and
wherein
the roughened surface is brought into contact with a site of nerve damage in the spinal cord,
whereby the roughened surface is phagocytosed by in vivo tissue cells,
mesenchymal stem cells are expressed on the roughened surface,
a capillary is newly formed along the roughened surface, and
nerve cells at the site of nerve damage in the spinal cord are regenerated using the roughened surface as a scaffold.

4. A brain damage repair sheet (excluding a sheet applied to a dura mater, a sheet for wrapping a brain blood vessel, or a sheet for wrapping a cerebral aneurysm), comprising:
a first surface including a roughened surface to be brought into contact with a site of brain damage and phagocytosed by in vivo tissue cells; and
a second surface disposed opposite to the first surface;
wherein
the roughened surface comprises polytetrafluoroethylene as a main component, and
wherein
the roughened surface is brought into contact with a site of brain damage within the arachnoid,
whereby the roughened surface is phagocytosed by in vivo tissue cells,
mesenchymal stem cells are expressed on the roughened surface,
a capillary is newly formed along the roughened surface, and
a tissue at the site of brain damage are repaired using the roughened surface as a scaffold.

5. A spinal cord damage repair sheet (excluding a sheet applied to a spinal dura mater), comprising:
a first surface including a roughened surface to be brought into contact with a site of spinal cord damage and phagocytosed by in vivo tissue cells; and
a second surface disposed opposite to the first surface;
wherein
the roughened surface comprises polytetrafluoroethylene as a main component, and
wherein
the roughened surface is brought into contact with a site of spinal cord damage within the arachnoid,
whereby the roughened surface is phagocytosed by in vivo tissue cells,
mesenchymal stem cells are expressed on the roughened surface,
a capillary is newly formed along the roughened surface, and
nerve cells at the site of spinal cord damage are repaired using the roughened surface as a scaffold.

6. The nerve damage repair sheet according to any one of claims 1 to 3,
wherein the roughened surface is formed by a surface of an ion-implanted layer.

7. The nerve damage repair sheet according to claim 6, wherein the ion-implanted layer is configured such that the in vivo tissue cells can infiltrate the interior thereof.

8. The nerve damage repair sheet according to any one of claims 1 to 3,
wherein the roughened surface comprises a depression approximately the same size as the cells that phagocytose the roughened surface.

9. The nerve damage repair sheet according to any one of claims 1 to 3,
wherein, when the roughened surface is placed in contact with in vivo tissue, a new tissue extends along the roughened surface from the in vivo tissue, and the sheet is configured such that the new tissue interlocks with the irregularities of the roughened surface.

10. The nerve damage repair sheet according to any one of claims 1 to 3,
wherein, when the roughened surface is placed in contact with in vivo tissue for six months or more, the opaque polytetrafluoroethylene is configured to become transparent or semi-transparent.
